(19) Europäisches Patentamt
European Patent Office
Office européen des brevets

(11) EP 0 510 085 B1

(12) **EUROPEAN PATENT SPECIFICATION**

(45) Date of publication and mention
of the grant of the patent:
**08.09.1999 Bulletin 1999/36**

(21) Application number: **91903073.4**

(22) Date of filing: **10.01.1991**

(51) Int Cl.$^6$: **C12Q 1/68**, C07H 15/12,
C12N 15/00

(86) International application number:
**PCT/US91/00213**

(87) International publication number:
**WO 91/10746 (25.07.1991 Gazette 1991/17)**

(54) **DNA-DEPENDENT RNA POLYMERASE TRANSCRIPTS AS REPORTER MOLECULES FOR SIGNAL AMPLIFICATION IN NUCLEIC ACID HYBRIDIZATION ASSAYS**

DNS-ABHÄNGIGE RNS-POLYMERASE-TRANSKRIPTE ALS REPORTERMOLEKÜLE ZUR SIGNALVERSTÄRKUNG IN NUKLEINSÄURE-HYBRIDISIERUNGSUNTERSUCHUNGEN

TRANSCRIPTIONS D'ARN-POLYMERASE DEPENDANTE D'ADN SERVANT DE MOLECULES REPORTEES POUR L'AMPLIFICATION DE SIGNAUX DANS LES ANALYSES D'HYBRIDATION D'ACIDE NUCLEIQUE

(84) Designated Contracting States:
**AT BE CH DE DK ES FR GB GR IT LI LU NL SE**

(30) Priority: **10.01.1990 US 463022**

(43) Date of publication of application:
**28.10.1992 Bulletin 1992/44**

(73) Proprietor: **Chiron Diagnostics Corporation
East Walpole, MA 02032 (US)**

(72) Inventor: **URDEA, Michael, S.
Alamo, CA 94507 (US)**

(74) Representative: **Goldin, Douglas Michael et al
J.A. KEMP & CO.
14 South Square
Gray's Inn
London WC1R 5LX (GB)**

(56) References cited:

| | |
|---|---|
| **EP-A- 0 369 775** | **WO-A-89/03891** |
| **WO-A-89/06700** | **WO-A-90/01068** |
| **WO-A-90/02819** | **WO-A-90/13667** |
| **WO-A-91/17442** | **US-A- 4 786 600** |

- **DATABASE WPIL, week 9026, AN=90-198034, Derwent Publications Ltd, London, GB; & JP-A-02 131 599 (TORAY IND., INC.) 21-05-1990**
- **Proceedings of the National Academy of Sciences, Vol. 77, issued July 1980, SAITO et al., "Nucleotide Sequence of the Primary origin of Bacteriophage T7 DNA Replication: Relationship to Adjacent Genes and Regulatory Elements", pages 3917-3921, See entire document.**
- **Biochemistry, Vol. 20, issued 1981, OSTERMAN et al, "T7 Ribonucleic Acid Polymerase-Promoter Interaction", pages 4884-4892, see entire documents.**
- **Nucleic Acids Research, Vol. 16, No. 17, issued 1988, PETTY, "A Plasmid Vector for Cloning Directly at the Transcription Initiation Site of a Bacteriophage T7 Promoter", page 8738, see entire document.**
- **Proceedings of the National Academy of Sciences, Vol. 86, issued February 1989, KWOH et al., "Transcription-Based Amplification system and Detection of Amplified Human Immunodeficiency Virus Type I with a Bead-Based Sandwich Hybridization Format", pages 1173-1177, see the entire document.**

EP 0 510 085 B1

**Description**

Background of the Invention

Technical Field

This invention pertains to the detection and quantification of biomolecules by hybridization assay, and pertains more particularly to hybridization assays wherein reporter molecules are used for signal amplification.

Background

Nucleic acid hybridizations are now commonly used in genetic research, biomedical research and clinical diagnostics to detect and quantify particular nucleotide sequences which are present in heterogeneous mixtures of DNA, RNA, and/or other materials. In the basic nucleic acid hybridization assay, single-stranded analyte nucleic acid (either DNA or RNA) is hybridized, directly or indirectly, to a labeled nucleic acid probe, and the duplexes containing label are quantified. Both radioactive and nonradioactive labels have been used.

The basic assay lacks sensitivity. When the analyte is present in low copy number or dilute concentration the signal cannot be distinguished from the background noise. Variations of the basic scheme have been developed to facilitate separation of the target duplexes from extraneous material and/or to amplify the analyte sequences in order to facilitate detection, but these variations have suffered generally from complex and time consuming procedures, high background, low sensitivity, and difficulty in quantification. A primary object of the present invention is to provide an amplifier for use in hybridization assays that provides a highly reproducible gain in signal, a highly reproducible signal-to-noise ratio, is itself quantifiable and reproducible, and is capable of combining specifically with an analyte present at low concentration and with a "universal" reporter moiety to form a stable complex.

Commonly owned US Patent No. 4,868,105, issued 19 September 1989, describes a solution phase hybridization sandwich assay in which the analyte nucleic acid is hybridized to a "labeling probe" and to a "capturing probe". The probe-analyte complex is coupled by hybridization to a solid-support. This permits the analyte oligonucleotide to be removed from solution as a solid phase complex, thereby concentrating the analyte, facilitating its separation from other reagents, and enhancing its subsequent detection.

PCT Application WO 84/03520 and EPA 124221 describe a DNA hybridization assay in which: (1) analyte is annealed to a single-stranded DNA probe that has a tail that is complementary to an enzyme-labeled oligonucleotide, and (2) the resulting tailed duplex is hybridized to an enzyme-labeled oligonucleotide. The Enzo Biochem "Bio-Bridge"™ labeling system appears to be similar to the system described in these two patent applications. The "Bio-Bridge" system uses terminal deoxynucleotide transferase to add unmodified 3'-poly T-tails to a DNA probe. The poly T-tailed probe is hybridized to the target DNA sequence and then to a biotin-modified poly A.

EPA 204510 describes a DNA hybridization assay in which analyte DNA is contacted with a probe that has a tail, such as a poly dT-tail, and an amplifier strand that has a sequence, e.g., a poly dA sequence, that hybridizes to the tail of the probe and is capable of binding a plurality of labeled strands.

The main problem with these prior hybridization assays is that they lack sufficient specificity and/or signal to be useful for detecting very low levels of analyte.

Another commonly owned EP Application No. 88309697.6 (publication No. 0317077), filed 17 October 1988, and corresponding published International Application WO 89/03891 describe linear and branched oligonucleotides which can be used as a signal amplifier in hybridization assays. Here the amplifier oligomer has two domains--a first domain which is complementary to a target sequence (either the analyte per se or a "linker probe") and a second domain, present in repeating units, complementary to a labeled reporting sequence. The multiplication of reporting sequences per target sequence provides for the amplification of the signal. Commonly owned PCT Application WO90/13667, which has an earlier priority date, but was not published until 15th November 1990, describes sandwich DNA hybridization assays employing capture probes immobilized on a solid support and amplifier probes each comprising a first segment complementary to the target and a second segment complementary to an oligonucleotide unit of a nucleic acid multimer. In such assays, the nucleic acid multimer is again employed as above to also bind a labelled oligonucleotide.

Another approach has been to use nucleic acid polymerases to amplify target sequences. For example, the so-called polymerase chain reaction (PCR), uses repeated cycles of DNA primed, DNA-directed DNA polymerase synthesis to amplify sequences of interest (Saiki, R.K., et al., Science (1986) 230:1350-1354). The amplified target is then detected using the basic hybridization assay protocol.

RNA polymerases have also been used to amplify target sequences (Krupp, G., and Soll, D. FEBS Letters (1987) 212:271-275). This approach has been incorporated into a hybridization format that involves production of a double-stranded copy of the target sequence, insertion of a RNA polymerase promoter sequence, transcription of the copy and detection by hybridization assay (Kwoh, D.Y., et al., <u>Proc. Natl. Acad. Sci. U.S.A.</u> (1989) <u>86</u>:1173-1177). Since DNA-directed RNA polymerases produce up to $10^3$ copies of RNA per copy of DNA template, fewer cycles of amplification are required. Bacteriophage DNA-dependent RNA polymerases (e.g., T3, T7, SP6) have previously been employed for the preparation <u>in vitro</u> of specific RNA sequences from cloned or synthetic oligonucleotide templates and are well understood (Melton, D.A., et al., <u>Nucleic Acids Res.</u> (1984) <u>12</u>:7035-7056); Chamberlin, M. and Ryan, T., (1982) in "The Enzymes," Boyer, P.D., ed., <u>15</u>:87-108; Martin, C.T., and Coleman, J.E., <u>Biochemistry</u> (1987) <u>26</u>: 2690-2696). These polymerases are highly promoter specific. DNA sequences from 17 T7 promoters are known and a consensus sequence has been deduced (Oakley, J.L., and Coleman, J.E., <u>Proc. Natl. Acad. Sci. U.S.A.</u> (1977) <u>74</u>: 4266-4270; Dunn, J.J., and Studier, F.W., <u>J. Molec. Biol.</u> (1983) <u>166</u>:477-535). It is also known that to retain polymerase activity, only the promoter region must be double-stranded (Milligan, J.F., et al., <u>Nucleic Acids Res.</u> (1987) <u>15</u>: 8783-8799).

Finally, RNA-directed RNA polymerase has also been used to detect target sequences (Lizardi, P.M., et al., <u>Bio/technology</u> (1988) <u>6</u>:1197-1202; Lomeli, H., et al., <u>Clin. Chem.</u> (1989) <u>35</u>:1826-1831). In this system an RNA probe is prepared by coupling RNA complementary to the target sequence with RNA (MDV-1) (U.S. Patent No. 4,786,600) which serves as an exclusive template for the bacteriophage Q-beta (Q) replicase. First, the target is immobilized on a solid substrate, then the RNA probe is hybridized to the target and finally the probe is eluted. Subsequent addition of Q-beta-polymerase to the probe generates multiple copies of the template/target RNA. In a related assay, MDV-1 RNA was first bound to biotin, then coupled to an avidinylated target, and subsequently assayed as described above (Chu, B.C.F., et al., <u>Nucleic Acids Res.</u> (1986) <u>14</u>:5591-5603).

The use of Q-beta-replicase in hybridization assays has four major disadvantages:

  1) Q-beta-replicase is typically contaminated with MDV-1 RNA. Consequently, this system has very high background (poor signal-to-noise ratio) when the reporter sequence is the MDV-1 sequence itself;
  2) The probe is RNA. RNA is highly sensitive to degradation from the RNAase activity which is ubiquitous in crude cellular preparations, and from the alkaline conditions required to denature double-stranded DNA targets;
  3) Due to the secondary structure of MDV-1 RNA there is considerable nonspecific binding in hybridization assays, thus significantly lowering the sensitivity of the assay and precluding accurate quantification; and,
  4) The amount of signal (the RNA product of Q-beta-replicase) varies with the log of the number of probes originally bound to the target. Thus, this assay can only detect order-of-magnitude differences between the concentrations of analyte in various samples.

The invention disclosed herein has several advantages over the Q-beta-replicase method. First, the probe is DNA rather than RNA. Second, the assay has very high signal to noise ratio and very high sensitivity. Third, since the signal is amplified rather than the target, the oligomer which is actually measured will always have the same sequence and size, thereby enabling the standardization and optimization of assay conditions (in addition, most of the biological reagents can be used universally thereby further simplifying and standardizing the assay). Finally, the target can be easily and accurately quantified.

Published International Application No. WO90/02819, which has an earlier priority date, but was not published until 22nd March 1990, describes methods for detecting a target nucleic acid employing a probe comprising two domains: (1) an oligonucleotide capable of hybridizing to the target and (2) a single-stranded DNA sequence comprising a promoter sequence. In these methods, after hybridization of the probe to the target, hybridized probe is released from the target or the promoter sequence is released by cleavage. In either case, the next step is hybridization of a second oligonucleotide to form a double-stranded functional promoter joined to a single-stranded template for a replicatable RNA.

Published International Application No. WO 89/06700 discloses a method for detecting a target nucleic acid sequence in which a first oligonucleotide primer linked to a promoter sequence is employed together with a second oligonucleotide primer to provide after polymerase chain extension reactions a construct comprising a functional promoter joined to a double-stranded template region containing the target sequence.

<u>Summary of the Invention</u>

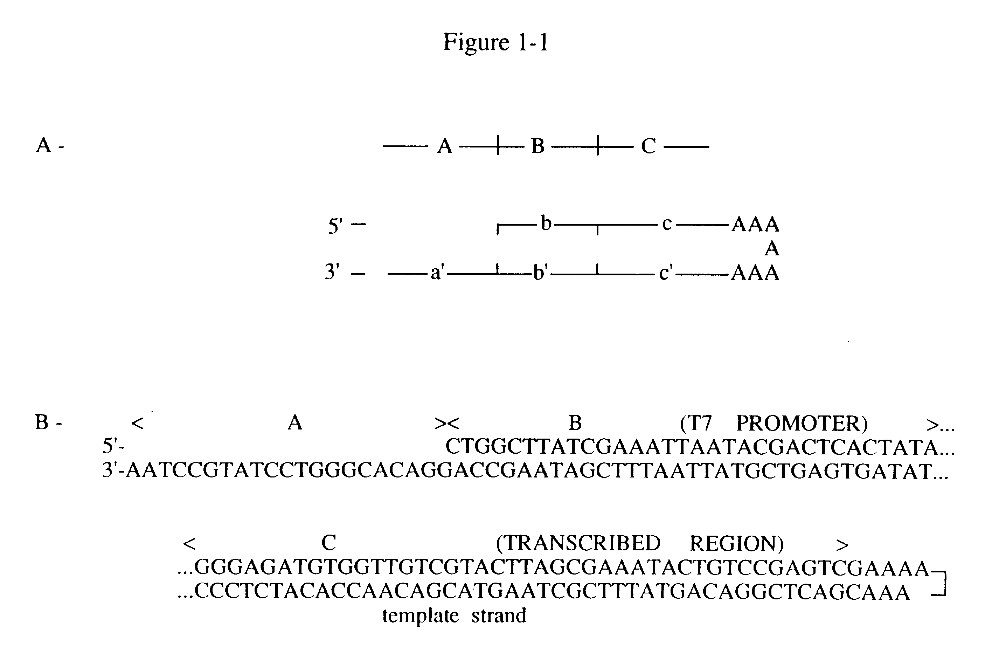

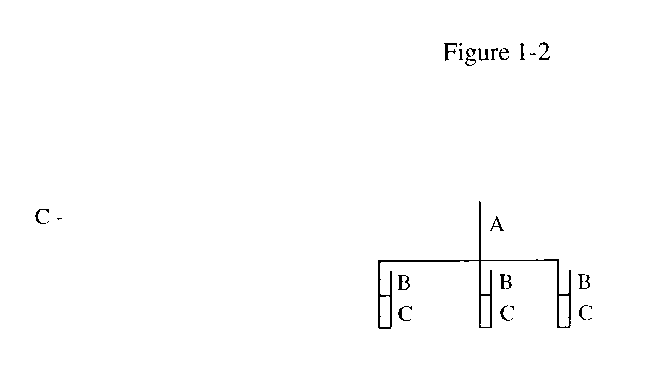

One aspect of the invention is a polydeoxynucleotide construct (template probe) for use as a signal amplifier in hybridization assays. The template probe is comprised of three domains as depicted in Figure 1A:

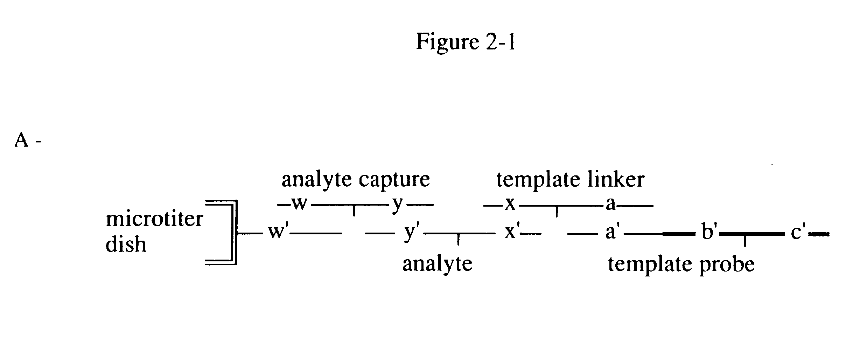

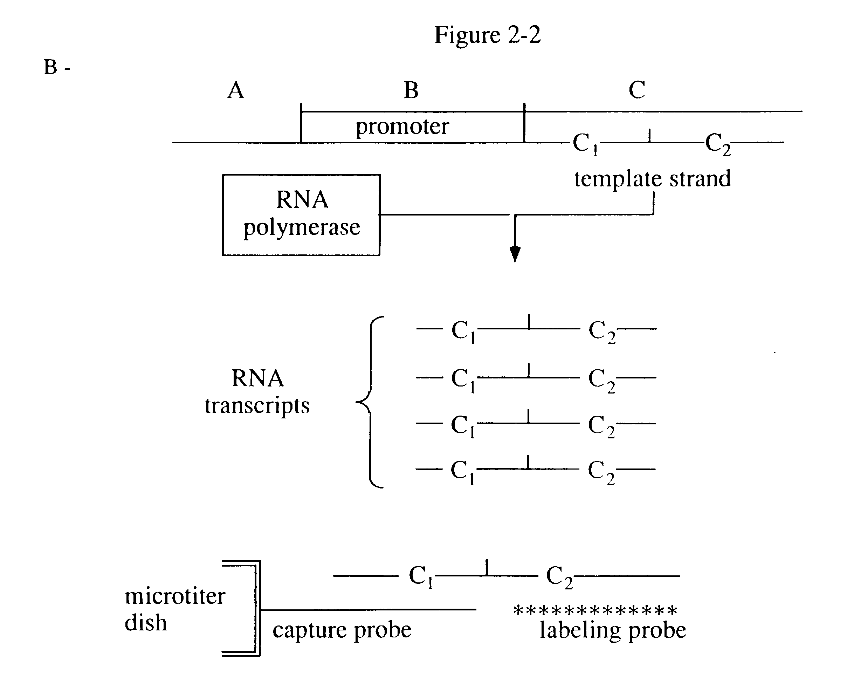

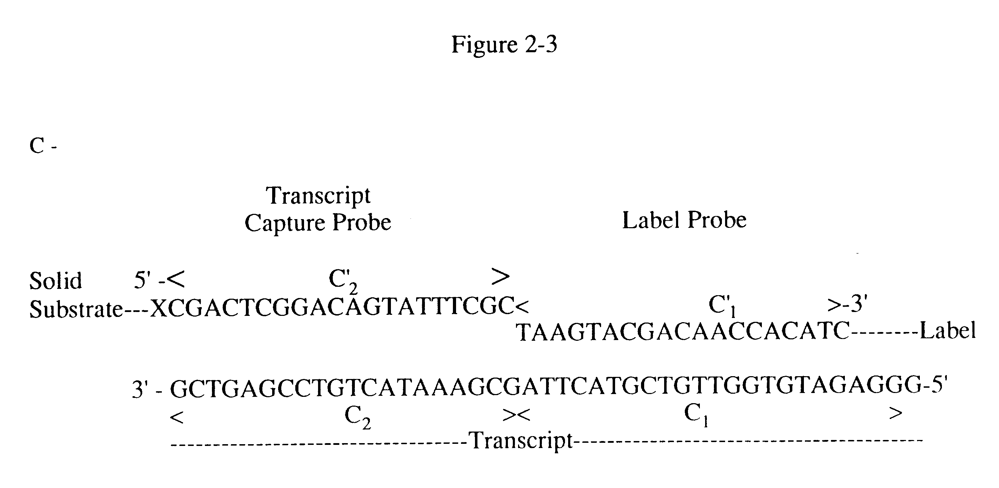

  (i) a first domain (A) which is single-stranded and has a nucleotide sequence (a') complementary to a target sequence (a) (Figure 2A) the target sequence comprising a domain either within the analyte sequence or within the sequence of an oligonucleotide which also contains a sequence domain complementary to the analyte sequence;
  (ii) a second domain (B) which is double-stranded and capable of function as a promoter for a DNA-dependent RNA polymerase enzyme activity; and

(iii) a third domain (C) which is either single- or double-stranded and adjacent to the second domain, such that the third domain is capable of functioning as a template (c') for the promoter activity of the second domain (Figure 2B).

A second aspect of the invention is a method of amplifying the biological signal used to detect and quantify an oligonucleotide, or other biomolecular analyte, in a hybridization assay comprising the following steps:

(i) immobilizing the analyte, directly or indirectly, on a solid substrate; and hybridizing the polydeoxynucleotide template probe described supra, directly or indirectly, to the analyte;
(ii) next removing the unhybridized template probe;
(iii) next transcribing (via a DNA-dependent RNA polymerase activity) multiple copies of RNA oligomers (c) which are complementary to the template sequence (c') of domain C of the amplifier; and
(iv) finally quantifying the RNA transcripts.

Brief Description of the Drawings

Figure 1A is a schematic representation of a monomeric template probe. Capital letters designate domains, and lower case letters designate strands within a domain. A primed letter designates a lower strand (read 3'- to 5'-, left to right). The a' sequence is complementary to a target sequence. The B domain is the promoter for a RNA polymerase. The c' sequence is the template for the RNA polymerase. The probe is synthesized as a single strand. The AAAAAAA represents the poly-A linker added to allow for self-annealing.

Figure 1B is the DNA sequence of one embodiment of the template probe. The promoter domain, B, consists of the consensus sequence of the bacteriophage T7 promoter (5'-TAATACGACTCACTATA-3') plus 15 additional residues 5' to the promoter sequence.

Figure 1C is a schematic representation of a multimeric template probe in which the double-stranded regions are self-annealing.

Figure 2A is a schematic representation of a sandwich hybridization assay system which incorporates the template probe. The analyte is indirectly immobilized upon a solid substrate by hybridization to the analyte capture probe and indirectly joined to the template probe via the template linker probe. "w'" represents the sequence of a region of the immobilized polynucleotide which is complementary to a region (w) of the analyte capture probe. "y" represents the sequence of a region of the analyte capture probe which is complementary to a region (y') of the analyte. "x'" represents the sequence of a region of the analyte which is complementary to a region (x) of the template linker probe. "a" represents a sequence of the template linker probe which is complementary to a', the sequence of the A domain of the template probe.

Figure 2B is a schematic representation of the use of RNA polymerase transcripts as reporter molecules in a hybridization assay. After hybridization of the analyte and template probe, an RNA polymerase is added and multiple RNA transcripts complementary to the template sequences (c) are produced. These sequences have two sub-domains: $c_1$ which is complementary to a capture probe immobilized upon a solid substrate; and $c_2$ which is complementary to a labeling probe. This allows for indirect immobilization of the label and easy quantification of the hybridization assay signal. "*" designates the incorporated label which may be radioactive, chemiluminescent, fluorescent or enzymatic.

Figure 2C is the DNA sequence of a transcript of the C domain, as well as the sequence of the transcript capture probe ($C_1$'), and the labeling probe ($C_2$'). X represents the $N^4$ methyl deoxycytidine derivative, [(6-aminocaproyl)-2-aminoethyl]-5-methyl-2'-deoxycytidine, used to couple the capture probe to the solid phase.

Figure 3A depicts the preparation of pII template probe.

Figure 3B depicts the preparation of pII$_L$ template probe.

Figure 3C depicts the preparation of pII$_R$ template probe.

Figure 4 depicts the domain A oligomer of Example 9 and the DNA sequences of oligo N, oligo S and oligo H.

Figure 5 depicts a protocol for a nucleic acid assay utilizing the T7 template probe and also utilizing an amplifier probe to further increase sensitivity and amplification of the signal.

Figure 6 is a graph showing the relative sensitivity and signal amplification of various template probes in assays for the presence of HIV in human serum.

Detailed Description of the Invention

A "biological signal" is a biochemically transmitted indicium of the occurrence of an event or presence of a specific molecule.

"DNA-dependent RNA polymerase" is an enzyme which facilitates the polymerization of RNA of specific sequence from a complementary DNA template.

A "domain" is a particular region of a polynucleotide characterized by its function.

An "immunological reaction" is the specific recognition and binding of an antibody to its corresponding epitope.

A "polydeoxynucleotide" is a polymeric DNA molecule. A "polynucleotide" is a polymeric DNA or RNA molecule.

A "promoter" is the site on a polydeoxynucleotide to which a RNA polymerase enzyme binds preparatory to initiating transcription.

"RNA-dependent RNA polymerase" is an enzyme which facilitates the polymerization of RNA of specific sequence from a complementary RNA template.

"Transcription" is a process, mediated by an enzyme, by which RNA is formed corresponding to a complementary polynucleotide template.

The "upper strand" of a double-stranded DNA molecule is the strand whose 5'-end is on the left as the sequence is read from left to right. The sequence of this strand is always presented above the sequence for its complementary "lower strand" which is read 3'- to 5'-, left to right.

Modes for Carrying Out the Invention

1. Template Probe

In one aspect of this invention a DNA probe (referred to as a "template probe") containing three functional domains has been designed in order to enhance the signal in hybridization assays.

The first domain ("A" in Figure 1A), has a sequence (a') usually 10 to 40 nucleotides in length, preferably 15 to 30 nucleotides, is single-stranded and is designed to hybridize to a complementary target sequence (a). In order to achieve hybrid stability, this domain will normally have a GC content in the range of 40% to 60%. The target sequence may subsist within the overall sequence of the polynucleotide to be assayed (referred to as the analyte) or it may subsist within an oligonucleotide linker which also has homology to the analyte. In a preferred embodiment, the analyte will be immobilized upon a solid substrate to facilitate subsequent washing procedures. This immobilization may be direct (e.g., polynucleotide preparations containing the analyte might be bound to a nitrocellulose filter) or indirect (e. g., a linker might be immobilized on the filter and the analyte subsequently hybridized to the linker).

The second domain (B), usually 10 to 40 basepairs in length, preferably 20 to 35 nucleotides, more preferably 30 to 35 nucleotides, is double-stranded and functions as a DNA-directed RNA polymerase promoter. This promoter is usually, derived from the promoter sequence of a bacteriophage, preferably any of the phage T3, T7, or SP6, more preferably from bacteriophage T7. This class of RNA polymerases is highly promoter specific. The T7 promoter is probably the best characterized. DNA sequences from 17 T7 promoters are known and a consensus sequence had been deduced: 5'-TAATACGACTCACTATA-3' (Oakley and Coleman; Dunn and Studier). Sequences 3' to the promoter on the complementary strand (the c' segment, whose 3' end is adjacent to the 5' end of the b' segment) serve as the template for transcription and the transcription of many template sequence variations can be accommodated. Only the promoter region itself must be double-stranded (Milligan et al.).

Additional sequences may be added at the 5' end of the promoter. For example, in a preferred embodiment, the B region consists of the consensus sequence of the T7 promoter plus additional bases 5' to the consensus sequence which are identical to the sequence of the pT7 plasmids (available from US Biochemicals) up to the PvuII restriction site (Figure 1B). These sequences may or may not have an effect on transcription.

The third domain (C) is directly 3' to the second domain and the c' strand of this domain serves as the template for the domain B promoter. Domain C may be as small as 30 nucleotides in length, or as long as 10 Kb. In a preferred embodiment the domain is 40 to 45 bases. In another preferred embodiment the domain is 3.4 Kb and is substantially similar to the genomic DNA of Hepatitis B virus. This domain may be either single- or double-stranded, and the 3' end of the c' template strand (directly adjacent to the promoter) usually is a cytosine residue.

The proper 5' to 3' relation of the promoter (B domain) to the template (C domain) is necessary for proper transcription of the template. The promoter is directly 5' to the template and the template is read 3' to 5'. However, it will be appreciated by those skilled in the art that the orientation of the B/C domains to the A domain is not critical. Thus template probes constructed as domains A-B-C, or as B-C-A will produce the same transcript and therefore may be constructed in either form.

The RNA transcription product (c) of the C domain functions as a reporter molecule for the presence and quantity of analyte. Signal amplification occurs because each template produces $10^1$ to $10^4$ transcripts. The sequence of this domain may be designed with a random sequence, evaluated by computer analysis to minimize the possibility of cross-reaction with other probes in the system, or alternatively, may be a known sequence which has been specifically chosen.

Further amplification can be achieved by designing the template probe with multimeric promoter/template (B/C) domains (Figure 1C). These multimeric units may be either in a linear array or branched molecules. For further details concerning the technology for the production and application of such multimers in hybridization assays, see EPA publication No. 0317077.

In a multimeric template probe the total number or repeating B/C units will usually be in the range of 2 to 200, more usually 5 to 20. The B/C units of the multimer may be covalently linked directly to each other through phosphodiester bonds or through interposed linking agents such as nucleic acid, amino acid, carbohydrate or polyol bridges, or through other cross-linking agents that are capable of cross-linking nucleic acid strands. The site(s) of linkage may be at the ends of the unit (in either normal 3'-5' orientation or randomly) and/or at one or more internal nucleotides in the strand. In linear multimers the individual units are linked end-to-end to form a linear polymer. In branched multimers three or more oligonucleotide units emanate from a point of origin to form a branched structure. The point of origin may be another oligonucleotide unit or a multifunctional molecule to which at least three units can be covalently bound. The multimer may be totally linear, totally branched, or a combination of linear and branched portions. Preferably there will be at least two branch points in the multimer, more preferably at least three. The multimer may include one or more segments of double-stranded sequences.

Template probes may be prepared by cloning, enzymatic assembly, chemical cross-linking techniques, direct chemical synthesis or a combination thereof. When prepared by cloning, nucleic acid sequences that encode the entire probe or fragments thereof can be made in single- or double-stranded form by conventional cloning procedures. When made in double-stranded form, the probe is denatured to provide single strands. Template probes may also be cloned in single-stranded form using conventional single-stranded phage vectors such as M13.

The A domain is single-stranded, the B domain is double-stranded, and the C domain may be either single- or double-stranded. A particular domain (e.g., B domain) can subsequently be made double-stranded by hybridization with its complementary strand--cloned separately. Alternatively, the entire template probe can be cloned as a single-stranded, self-annealing polynucleotide (a' b' c' c b). In this case four to ten additional nucleotides, preferably 5-7 nucleotides, are added to the sequence as a spacer between c and c' to allow for proper contouring of the double-stranded region when it is self-annealed. The spacer is usually poly-A, but may be modified to minimize hybridization cross-reactivity between various probes in the assay.

If multimeric probes are desired, fragments are linked enzymatically or chemically to form the multimer. When assembled enzymatically, the individual units are ligated with a ligase such as T4 DNA ligase. When prepared by chemical cross-linking, the individual units may be synthesized with one or more nucleic acids that have been derivatized to have functional groups that provide linking sites or derivatized after the oligonucleotide has been synthesized to provide such sites. A preferred procedure for chemical cross-linking is to incorporate $N^4$-modified cytosine bases into the nucleotide as described in the commonly owned EPA publication No. 0225807.

When prepared by direct chemical synthesis oligonucleotides containing derivatized nucleic acids whose functional groups are blocked are made by conventional oligonucleotide synthesis techniques. The functional groups are unblocked and oligonucleotide units are synthesized out from the unblocked site(s).

## 2. Amplified Hybridization Assay

Another aspect of this invention employs template probes in hybridization assays. The analyte may be any nucleotide sequence of interest--either DNA or RNA. The analyte sequence may constitute an entire molecule or only a portion of a molecule. The analyte may be a homogeneous polynucleotide, present in low concentration in a prepared sample or it may be a minority species in a heterogeneous mixture of polynucleotides. The analyte may also be from a variety of sources, e.g., biological fluids or tissues, food stuffs, environmental materials, etc., or it may be synthesized in vitro.

The analyte may be prepared for the hybridization analysis by a variety of means, e.g., proteinase K/SDS, chaotropic salts, etc. Also, it may be of advantage to decrease the average size of the analyte by enzymatic, physical or chemical means, e.g., restriction enzymes, sonication, chemical degradation (e.g., metal ions), etc. The fragments may be as small as 0.1 kb, usually being at least about 0.5 kb and may be 1 kb or higher. Where the analyte sequence is lengthy, for example a viral genome, several different regions of the analyte may be used as targets of an analyte probe.

The analyte sequence is provided in single-stranded form for analysis. Where the sequence is naturally present in single-stranded form, denaturation will not usually be required. However, where the sequence is present in double-stranded form, the sequence will be denatured. Denaturation can be carried out by various techniques, such as alkali, generally from about 0.05 to 0.2 M hydroxide, formamide, chaotropic salts, heat, or combinations thereof.

In a first step, the analyte may be immobilized directly upon a solid phase or by sandwich hybridizations in which the analyte is bound to an oligonucleotide that is in turn bound to a solid phase. A particularly useful approach is a solution phase sandwich hybridization described in commonly owned EPA publication No. 0225807.

In a sandwich hybridization assay with a capture step the template probe is used as follows: Single-stranded analyte nucleic acid is incubated under hybridization conditions with an excess of two single-stranded nucleic acid probes, (1) an analyte capture probe having a first binding sequence complementary to the analyte and a second binding sequence that is complementary to a single-stranded oligonucleotide bound to a solid phase, and (2) a template linker probe having a first binding sequence that is complementary to the analyte and a second binding sequence that is complementary to domain A of the template probe.

In a preferred embodiment, a set of analyte capture probes may be used wherein each member of the set has a different first binding sequence complementary to a different segment of the analyte while all members of the set have the same second binding sequence. Similarly, a set of template linker probes may be used wherein each member of the set has a different first binding sequence complementary to a different segment of the analyte, but all members of the set have the same second binding sequence complementary to domain A of the template probe. This approach has the advantage of enabling the simultaneous detection of closely related variants of an analyte, e.g. the genomes of related viral strains.

By using analyte capture and template linker probes, the solid matrix and the template probe can be used as a "universal" reagent and different immobilized oligonucleotide matrices and template probes need not be made for each analyte.

Usually, hybridization conditions consist of an aqueous medium, particularly a buffered aqueous medium, which includes various additives. These additives include the polynucleotides to be hybridized, salts (e.g., sodium citrate 0.017M to 0.17M and sodium chloride 0.17M to 1.7M), nonionic or ionic detergents (0.1 to 1.0%), and carrier nucleic acids. Nonaqueous solvents such as dimethylformamide, dimethylsulfoxide, and formamide may also be used. The mixture is incubated for 15 to 75 minutes at 45°C to 70°C. The stringency of the hybridization is regulated by temperature and salt concentration and may be varied depending on the size and homology of the sequences to be hybridized. For hybridization of sequences to bound DNA, the empirical formula for calculating optimum temperature under standard conditions (0.9 M NaCl) is:

$$T(°C) = 4(N_G + N_C) + 2(N_A + N_T) - 5°C,$$

where $N_G$, $N_C$, $N_A$, and $N_T$ are the percentage of G, C, A, and T bases in the sequence (Meinkoth, J., et al., Anal. Biochem. (1984) 138:267-284).

The resulting product is a three component nucleic acid complex of the two probes hybridized to the analyte by their first binding sequences. The second binding sequences of the template linker probe and analyte capture probe remain as single-stranded tails as they are not complementary to the analyte.

This complex is then added under hybridizing conditions to a solid phase having a single-stranded oligonucleotide bound to it that is complementary to the second binding sequence of the analyte capture probe. The resulting product comprises the complex bound to the solid phase via the duplex formed by the oligonucleotide bound to the solid phase and the second binding sequence of the analyte capture probe. The solid phase with bound complex is then separated from unbound materials and washed to remove any residual unbound material.

The template probe is then added to the solid phase-analyte-probe complex under hybridization conditions to permit the template probe to hybridize to the available second binding sequences of the template linker probe of the complex (the target sequence of the template probe). The resulting solid phase complex is then separated from any unbound template probe and washed.

Next, the RNA polymerase specific for the promoter region (domain B) of the template probe is added under appropriate transcription conditions and multiple RNA copies (c) of the C domain template (c') are produced. The amount of transcript is proportional to the quantity of the analyte in the initial preparation.

Transcription conditions consist of an aqueous medium, preferably a buffered aqueous medium, with appropriate salts, usually including a magnesium salt, a mixture of NTPs (rATP, rUTP, rGTP, rCTP), a RNA polymerase enzyme and usually include various denaturing agents, protein carriers, and RNAse inhibitors. Incubation is usually for 15 to 90 minutes, usually 60 minutes; and at a temperature which is optimal for the chosen enzyme, usually 35°C to 42°C, usually 37°C.

The sequence of the C domain is designed for a specific detection scheme and several such schemes may be employed to quantify the transcripts. For example, the transcription product (c) of the C domain may be subdivided into 2 subdomains--$c_1$ and $c_2$ (Figure 2B). Subdomain $c_1$ is complementary to a transcript capture probe which has been immobilized on a solid substrate. Subdomain $c_2$ is complementary to an amplifier probe. After hybridization the amount of label retained is linearly proportional to the amount of analyte present in the original sample. In a variation of this approach, the transcripts are sandwiched with linker probes, i.e., the transcript capture probe is in solution rather than immobilized, and contains a second domain which is complementary to an immobilized oligonucleotide; and subdomain $c_2$ is complementary to an amplifier linker probe which in turn is complementary to the amplifier probe. This sandwiching arrangement is similar to the use of analyte capture and template linker probes to sandwich the analyte as described above.

In an alternate embodiment the transcript of the C domain has only a $c_1$ subdomain. The C domain is transcribed in the presence of labeled ribonucleotide triphosphates and the labeled transcript is subsequently bound to an immobilized transcript capture probe through its complementary $c_1$ subdomain and quantified.

In yet another embodiment the transcript of the C domain has only a $c_2$ subdomain. The C domain is transcribed in the presence of biotinylated ribonucleoside triphosphates and the transcripts is captured on avidin beads. The transcript is then annealed to an amplifier probe through its complementary $c_2$ subdomain and quantified.

Several other methods of labeling and detecting the transcript of the amplifying probe are possible, including the simultaneous use of labeled ribonucleotides and avidin/biotin coupling, and will be obvious to those skilled in the art.

Capture, Linker and Amplifier Probes

The first binding sequences of the analyte capture probe and template linker probe are complementary to the analyte sequence. Similarly, the first binding sequences of the transcript capture and amplifier linker probes are complementary to the reporter transcripts. Each first binding sequence is at least 12 nucleotides (nt), usually at least 25 nt, more usually at least 30 nt, and not more than about 150, usually not more than about 75, preferably not more than about 50 nt. They will normally be chosen to bind to different sequences of the analyte. The first binding sequences may be selected based on a variety of considerations. Depending upon the nature of the analyte, one may be interested in a consensus sequence, a sequence associated with polymorphisms, a particular phenotype or genotype, a particular strain, or the like.

The second binding sequences of the analyte capture probe and template linker probe are selected to be complementary, respectively, to the oligonucleotide attached to the solid phase and to an oligonucleotide unit of the template probe and so as to not be encountered by endogenous sequences in the sample/analyte. The second binding sequence may be contiguous to the first binding sequence or be spaced therefrom by an intermediate noncomplementary sequence. The probes may include other noncomplementary sequences if desired. These noncomplementary sequences must not hinder the binding of the binding sequences or cause nonspecific binding to occur.

The capture probes and linker probes may be prepared by conventional oligonucleotide synthesis procedures or by cloning.

It will be appreciated that the binding sequences need not have perfect complementarity to provide homoduplexes. In many situations, heteroduplexes will suffice where fewer than about 10% of the bases are mismatches, ignoring loops of five or more numbers. Accordingly, as used herein the term "complementary" intends a degree of complementarity sufficient to provide a stable and specific duplex structure.

The solid phase that is used in the assay may be particulate or be the solid wall surface of any of a variety of containers, e.g., centrifugal tubes, columns, microtiter plate wells, filters, tubing, etc. Preferably, particles will be employed of a size in the range of about 0.4 to 200 micrometres, more usually from about 0.8 to 4.0 micrometres. The particles may be any convenient material, such as latex, or glass. The oligonucleotide that is complementary to the second binding sequence of the analyte capture probe may be stably attached to the solid surface through functional groups by known procedures.

It will be appreciated that one can replace the second binding sequence of the capture probe and the oligonucleotide attached to the solid phase with an appropriate ligand-receptor pair that will form a stable bond joining the solid phase to the first binding sequence of the capture probe. Examples of such pairs are biotin/avidin, thyroxine/thyroxine-binding globulin, antigen/antibody, carbohydrate/lectin, and the like.

The amplifier probes will include a sequence complementary to the $C_2$ subdomain of the transcripts of the template probe, or to a subdomain of an amplifier linker probe. The amplifier probe is capable of hybridizing to one or more labels or labeling probes which directly or indirectly provide for a detectable signal. The labels may be incorporated in individual residues of the complementary sequence or may be present as a terminal domain or terminal tail having a plurality of labels. Various means for providing labels bound to the sequence have been reported in the literature. See, for example, Urdea et al., Nucl. Acids Res. (1988) 4937; Leary et al., Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al., Nucl. Acids Res. (1985) 13:2399; Meinkoth and Wahl, Anal. Biochem. (1984) 138:267. The labels may be bound either covalently or noncovalently to the complementary sequence. Labels which may be employed include radionuclides, fluorescers, chemiluminescers, dyes, enzymes, enzyme substrates, enzyme cofactors, enzyme inhibitors, enzyme subunits, metal ions, and the like. Illustrative specific labels include fluorescein, rhodamine, Texas red, phycoerythrin, umbelliferone, luminol, NADPH, galactosidase, horseradish peroxidase, alkaline phosphatase, etc. See Urdea et al. for a comparison of nonradioisotopic labeling methods.

The labeling probes can be conveniently prepared by chemical synthesis

By providing for a terminal group which has a convenient functionality, various labels may be joined through the functionality. Thus, one can provide for a carboxy, thiol, amine, hydrazine or other functionality to which the various labels may be joined without detrimentally affecting duplex formation with the sequence. As already indicated, one can have a molecule with a plurality of labels joined to the sequence complementary to the labeling sequence. Alternatively, one may have a ligand bound to the labeling sequence and use a labeled receptor for binding to the ligand to provide the labeled analyte complex.

The ratio of analyte capture probe and template linker probe to anticipated moles of analyte will each be at least stoichiometric and preferably in excess. This ratio is preferably at least about 1.5:1, and more preferably at least 2:1. It will normally be in the range of 2:1 to 10,000:1. Concentrations of each of the probes will generally range from about $10^{-9}$ to $10^{-6}$ M, with sample nucleic acid concentrations varying from $10^{-21}$ to $10^{-12}$ M. The hybridization steps of the assay will generally take from about 10 minutes to 2 hours, frequently being completed in about 1 hour. Hybridization can be carried out at a mildly elevated temperature, generally in the range from about 20°C to 80°C, more usually from about 35°C to 70°C, particularly 65°C. Additional conditions for the hybridization reaction are described infra.

The procedure used in the separation steps of the assay will vary depending upon the nature of the solid phase. For particles, centrifugation or filtration will provide for separation of the particles, discarding the supernatant or isolating the supernatant. Where the particles are assayed, the particles will be washed thoroughly, usually from one to five times, with an appropriate buffered medium containing detergent, e.g., PBS with SDS. When the separation means is a wall or support, the supernatant may be isolated or discarded and the wall washed in the same manner as indicated for the particles.

Depending upon the nature of the label, various techniques can be employed for detecting the presence of the label. For fluorescers, a large number of different fluorometers are available. With enzymes, either a chemiluminescent, fluorescent or a colored product can be provided and determined fluorometrically, spectrophotometrically or visually. The various labels which have been employed in immunoassays and the techniques applicable to immunoassays can be employed with the subject assays.

In a hybridization assay in which the analyte nucleic acid is bound directly to a solid phase, such as a "dot blot" assay, the template probe is hybridized directly to the bound analyte. In these instances, the A domain of the template probe is complementary to a sequence of the analyte.

The template probe may also be used in other assays such as direct, indirect, and sandwich immunoassays and assays for ligand receptors, for instance cell surface receptors. In these instances, rather than a label, the reagent that plays the role of the labeled antibody, or other ligand which binds to the analyte (antigen or ligand receptor), has an attached oligonucleotide that is complementary to a', the sequence of the A domain of the template probe. For instance, in a sandwich immunoassay for an antigen analyte, the analyte sample is incubated with a solid phase to which is bound a first antibody to the antigen. Unbound sample is removed from the solid phase and a second antibody to the antigen, which is coupled to an oligonucleotide complementary to a', is reacted with the bound complex to form a three-membered complex. Following removal of excess second antibody the template probe is then hybridized to the complex via the oligonucleotide bound to the second antibody. Excess template probe is removed and RNA polymerase is added as described supra. Finally, the transcription product is quantified as described.

In an alternative embodiment, the template probe may be synthesized without an A domain and coupled directly to the ligand receptor or antibody by means of avidin/biotin or an equivalent stably bonding pair as described previously. The template probe may also be covalently attached to the ligand receptor by means of chemical synthesis.

Kits for carrying out amplified nucleic acid hybridization assays according to the invention will comprise in packaged combination the following reagents: the template probe; the appropriate DNA-directed RNA polymerase; an appropriate labeling probe; a solid phase that is capable of binding to the analyte; optionally an analyte capture probe if the assay format is one in which the analyte is bound to the solid phase through an intermediate oligonucleotide or other ligand; and optionally a template linker probe if the assay format is one in which the template probe is not hybridized directly to the analyte. Similarly, these kits may also contain transcript capture probes amplifier probes and amplifier linker probes. These reagents will typically be in separate containers in the kit. The kit may also include a denaturation reagent for denaturing the analyte, hybridization buffers, wash solutions, negative and positive controls and written instructions for carrying out the assay.

EXAMPLES

Example 1

A. T7 Template Probe.

The probe was designed as shown in Figure 1A. The a' sequence of the A domain is shown in Figure 1B and is complementary to the a region of the template linker probe depicted in Figure 2A. The sequence of the promoter domain, B, (shown in Fig. 1B) contained the consensus T7 promoter sequence plus 15 additional bases 5' to the promoter and identical to the sequence of the pT7 plasmid (available from US Biochemicals) up to the PvuII restriction site. The additional 15 basepairs may be extraneous; however, they were incorporated since the initial experiments conducted with template probes that had been cloned into the pT7 vector proved successful. Thus, even template probes made by chemical synthesis have retained this plasmid portion. The C domain was designed as a random sequence. It was evaluated by computer analysis to minimize potential hybridization cross-reactivity with other probes in the system.

B. T3 Template Probe.

The probe is designed as in Example 1A, above, except that the consensus sequence for the DNA-directed RNA polymerase promoter sequence of bacteriophage T3 (5'-TATTAACCCTCACTAAA-3') is substituted for the consensus sequence for the T7 promoter (5'-TAATACGACTCACTATA-3').

C. SP6 Template Probe.

The probe is designed as in Example 1A, above, except that the consensus sequence for the DNA-directed RNA polymerase promoter sequence of bacteriophage SP6 (5'-ATTTAGGTGACACTATA-3') is substituted for the consensus sequence for the T7 promoter and the first six 5' nucleotides of domain C are 5'-GAAGGG-3' rather than 5'-GGGAGA-3, as is the case in Example 1A.

Example 2

Hybridization Assay for the Pilin Gene DNA of Neisseria gonorrhoeae Using a Microtiter Dish Assav Procedure and the T7 RNA polymerase.

A. Standard Analyte DNA.

The N. gonorrhoeae strain 31707 from the Neisseria Reference Laboratory (Seattle, WA) was used. DNA was prepared from this strain, as well as from several nonpathogenic commensal strains of Neisseria used as controls, by the addition of a proteinase K/SDS solution as described in Urdea et al. (Gene (1987) 61:253).

B. Oligonucleotide Bound to Solid Support (Figure 2A).

A microtiter dish assay procedure was employed. Microtiter dishes were prepared as follows. Two types of microtiter dish wells were prepared: (1) N wells for sample work-up and negative controls, and (2) S wells for capture of the probe-analyte complex from samples and positive controls.

N wells were produced as follows: 300 $\mu$l of HM buffer (0.1% SDS, 4xSSC, 1 mg/ml sonicated salmon sperm DNA, 1 mg/ml poly A, 10 mg/ml BSA) was added to Immulon II Remov-a-wells (Dynatech Inc.). The well strips were covered and left standing at room temperature for 1 hour. The HM buffer was removed by aspiration and the wells were washed 3 times with 400 $\mu$l of 1 x PBS. The strips were covered with plastic wrap and stored at 4°C until used.

S wells were prepared from the Immulon II strips as follows. To each well, 200 $\mu$l of a 200 $\mu$g/ml solution of poly-phenylalanyl-lysine (Sigma Chemical Inc.) in water. The covered strips were left at room temperature for 30 min to 2 hr, then washed as above.

Next, a 21 base oligomer, 5'-XCACCACTTTCTCCAAAGAAG-3', where X represents the N4-(6-aminocaproyl-2-aminoethyl) derivative of 5-methyl cytidine, was synthesized according to the method of Warner et al. (DNA (1984) 3:401) and purified as described by Urdea et al., supra. The N4-modified cytosine base facilitates the chemical cross-linking of the oligonucleotide as described in commonly owned EPA Publication No. 0225807 and Urdea, M.S., et al., Nucl. Acids Res. (1988) 16:4937-4956.

A 10 OD sample of the synthesized oligonucleotide in 60 $\mu$l of 1 x PBS was treated with 140 $\mu$l of dimethyformamide containing 10 mg of ethylene glycol bis (succinimidylsuccinate) (Pierce Chemical Inc.). The mixture was vortexed and incubated in the dark at room temperature. After 15 min, the solution was passed over a Sephadex® G-25 column (PD-10 from Pharmacia), previously equilibrated with 30 ml of 1 x PBS. The void volume of the column was diluted to a final volume of 35 ml with 1 x PBS. To each well, a 50 $\mu$l aliquot of the oligonucleotide solution was added. After covering with plastic wrap, the wells were incubated at room temperature in the dark for 30 min to overnight. The wells were washed with 1 x PBS, then coated with HM buffer, washed, and stored as above.

C Analyte Capture Probes (Figure 2A).

A set of 3 single-stranded oligomers each having a varying 30 base long portion complementary to a specific sequence of the pilin gene and a constant 20 base long 5'-portion complementary to the oligonucleotide bound to the solid phase was synthesized by the automated phosphoramidite procedures described in Warner et al., supra, and purified by the method of Sanchez-Pescador and Urdea, supra. The sequences complementary to the pilin gene were based on the N. gonorrhoeae pilin sequence described by Bergstrom, S., et al. (PNAS USA (1986) 83:3890-3894). The 5' portions of the probes were complementary to segments of the pilin sequence and were as follows:

| **Probe Designation** | **5'-Sequence** |
|---|---|
| GCP-XT1-4 | GAT GTG GCG GGC GCG CGT TCA AAG GCT TCG |
| GCP-XT1-8 | GAG GCT GTA GTT TCC GTT TAT ACA ATT TCT |
| GCP-XT1-12 | GCC AAG CCA TTT TAC CAA GAC GCC TGT CGG |

The 3'-portion of each analyte capture probe was constructed to be complementary to the sequence of the oligonucleotide attached to the solid support described infra.

D. Template Linker Probes (Figure 2A).

A set of 12 single-stranded oligomers each consisting of a varying 30 base long portion complementary to a specific sequence of the pilin gene and a constant 20 base long 3'-portion complementary to the template probe (Figure 2A) were synthesized by the procedures of Warner et al., supra and purified according to Sanchez-Pescador and Urdea, supra.

The 5' portions of the probes were complementary to segments of the pilin sequence and were as follows:

| **Probe Designation** | **5'-Sequence** |
|---|---|
| GCP-LLA2C-1 | ATA CTT ATG GGA AGT TTT TCC GAA ATG GGA |
| GCP-LLA2C-2 | GCT CGA CTA CTA ACA CTA GCG ATA GCA GCC |
| GCP-LLA2C-3 | AAA CCG CAA TCA GCG GGA AGG GCG GAT GGT |
| GCP-LLA2C-5 | GGA AAA CCG GCT TCC AGT TTT TAG TCG GCA |
| GCP-LLA2C-6 | GCT CAT AAT GGA CTT AAG GCC GTT TAC CGG |
| GCP-LLA2C-7 | TTT GTT GTG AAG ACG GCC GCA CCG TAG GGG |
| GCP-LLA2C-9 | ACT TCA ATT TTT GCC GCA GCA ATG GCG GTG |
| GCP-LLA2C-10 | CGA AAG TTC GCC GCA TTT GTT ACT AAT GTT |
| GCP-LLA2C-11 | GTT TTT TGA GAG GGA CAC CCG GTC CGC ACT |
| GCP-LLA2C-13 | ATG CGC GTG GCT GCT GCT GTG GCA ACG GCT |
| GCP-LLA2C-14 | GTT TCT GCC GTT TCT TTA GCT GTG GTT CGT |
| GCP-LLA2C-15 | CGG CAG TTG GAC GGC GCT ATT CCG TAG ACT |

The 3'-portion of each template linker probe was constructed to be complementary to the sequence of the A domain of the template probe.

E. Labeled Oligomer (Figure 2B).

An 18 base oligomer, 5'-XGGTCCTAGCCTGACAGC-3', where X is defined as above, was synthesized as described, and combined with alkaline phosphatase (AP) as follows: Calf intestinal AP (3mg in buffer; immunoassay grade, Boehringer-Mannheim) was placed in a Centricon 30 Microconcentrator. Approximately 2 ml of 0.1 M sodium borate, pH 9.5, was then added and the device was spun at 3500 rpm until a final volume of 40 μl was obtained. The alkylamino oligonucleotide was then activated with p-phenylene diisothiocyanate (DITC; Pierce Chemicals) in 95:5 (v/v) dimethylformamide: 0.1 M sodium borate, pH 9.3, extracted with n-butanol, and combined with the protein. The final product was stored at 4°C. See Urdea et al. (Nuc. Acids Res. (1988) 16:4937).

F. Microtiter Dish Procedure.

For duplicate analyses, 20 μl of each sample was placed into 2 N wells, then treated with 25 μl of proteinase K/SDS solution. The wells were covered with a Linbro-Titertek™ microtiter plate sealer, gently agitated, and incubated at 65°C for 30 min in a water bath. The analyte capture and template linker probe sets in a 1 M NaOH were added in 10 μl to each well. After sealing, the samples were incubated for 10-30 min at 65°C to 72°C as above. The solutions were neutralized with 26 μl 0.38 M acetic acid (or 0.76 M 3-[N-morpholino] propane sulfonic acid (MOPS), free acid), 12.3 x SSC, then incubated for an additional 15-30 min covered at 65°C. From each N well, 40 μl of sample was transferred to a new S well containing the solid supported capture probe. The wells were sealed and set at 65°C for 1 hour. Each well was then washed 2 times by aspiration with 0.1% SDS, 0.1 x SSC. See Folberg et al. (Molec. and Cell. Probes (1989) 3:59).

The template probe was subsequently annealed to the complex by incubation of 100 fmoles of template probe in 40 μl of 4 x SSC with 100 μg/ml poly A at 55°C for 1 hr followed by two washes with 0.1 x SSC, 0.1% SDS and two washes with 0.1 x SSC.

Transcription of domain C was effected by incubating the complex in 20 μl of a solution containing 40 mM Tris HCl (pH 8), 20 mM $MgCl_2$, 10 mM NaCl, 1 mM Spermidine, 10 mM Dithiothreitol, 0.15 mg/ml Bovine Serum Albumin, 1.25 mM each of rATP, rCTP, rGTP, rUTP, 1600 units/ml RNasin, and 2000 units/ml T7 RNA polymerase. This mixture was incubated at 37°C for 1 hour. Transcription was terminated by addition of 20 μl of a solution containing 8 X SSC, and 0.2% SDS and the entire mixture was transferred to new wells containing an immobilized capture probe with $c_1'$ sequences. Capture of the domain C transcripts (Figure 2B) was effected by incubation at 55°C for 1 hour followed by two washes with 0.1 X SSC, 0.1% SDS.

The domain C transcripts were then labeled by addition of 50 fmol of enzyme-labeled probe ($c_2'$) in 40 μl of 4 X SSC, 100 μg/ml poly A for 15 min. at 55°C. Finally, the complex was washed twice with 0.1 X SSC, 0.1% SDS, followed by two washes with 0.1X SSC.

For AP detection, an enzyme-triggered dioxetane-based reaction (Schapp et al. Tet. Lett. (1987) 28: 1159-1162) and US Patent 4,857,652), available from Lumigen Inc., was employed. The detection procedure was as follows. For the labeling step 40 μl HM buffer with the AP probe was added to each well and the wells were incubated at 55°C for 15 min. The supernatant was removed and the wells were washed 2 x with 380 μl of 0.1 x SSC and 0.1% SDS. The wells were then washed 2 x with 380 μl of 0.1 x SSC to remove any remaining SDS. 20 μl of $3.3 \times 10^{-4}$ M dioxetane reagent in CTAB buffer was added to each well. The wells were tapped lightly so that the reagent would fall to the bottom and gently swirled to distribute the reagent evenly over the bottom. The wells were covered with the microtiter plate sealer and incubated in a 37°C oven for one hour. The wells were then read with a luminometer.

Results

Tests were carried out on N. gonorrhoeae bacterial cells (strain 31707) as well as nonpathogenic Neisseria controls according to the protocol of Example 2, above. Results are presented as a signal to noise ratio (S/N) representing the value of the sample versus the value of the control. Cell number was determined by cell viability. For comparison, tests were also carried out on the same samples using a branched 5-site comb-type amplification multimer described in EP-A-0 317 077.

| Cell Number | T7 Transcription Assay | | Multimeric Assay | |
|---|---|---|---|---|
| | Trial 1 | Trial 2 | Trial 1 | Trial 2 |
| $8.3 \times 10^5$ | 186.76±25.39 | 139.93±44.87 | 90.94±48.69 | 82.15±16.55 |
| $8.3 \times 10^4$ | 18.35± 3.60 | 8.35± 4.20 | 9.94± 8.71 | 14.56± 0.47 |
| $8.3 \times 10^3$ | 2.43± 0.37 | 1.55± 0.37 | 2.58± 1.40 | 2.55± 0.42 |

Example 3

Hybridization Assay Using the T3 RNA Polymerase

A hybridization assay is employed using the same protocol as in Example 2, supra, except that domain B of the template probe contains the sequences for the T3 RNA polymerase promoter rather than the T7 promoter. The sequence of the T3 promoter has been previously disclosed by Brown, J.E., et al. (Nucleic Acids Res. (1986) 14: 3521-3526). The T3 promoter sequence is 5'-TAT TAA CCC TCA CTA AAG GGA GA-3' and replaces the T7 promoter sequence 5'-TAA TAC GAC TCA CTA TAG GGA GA-3'.

Example 4

Hybridization Assay Using the SP6 RNA Polymerase

A hybridization assay is employed using the same protocol as in Example 2, supra, except that domain B of the template probe contains the sequence for the SP6 RNA polymerase promoter rather than the T7 promoter. The sequence of the SP6 promoter has been previously disclosed by Brown et al., supra. The SP6 promoter sequence is 5'-ATT TAG GTG ACA CTA TAG AAG GG-3' and replaces the T7 promoter sequence 5'-TAA TAC GAC TCA CTA TAG GGA GA-3'.

Example 5

Hybridization Assay for Hepatitis B Virus (HBV) DNA Using the Microtiter Dish Assay Procedure and T7 RNA Polymerase

DNA extracts of serum or plasma samples of patients potentially infected with hepatitis B virus (HBV) are prepared as described in copending US application No. 109,282 and are used as analyte as described in Example 2.
A set of single-stranded template linker probes, each having a varying 30 base long portion complementary to a specific sequence of the constant ds region of the HBV genome and a constant 20 base long 3'-portion complementary to the template probe used in Example 2 is synthesized by the procedures described in Example 2. The sequences of these probes are presented in Table 1 below.

## TABLE 1
## Template Linker Probes for HBV

| Probe Designation | Sequence |
|---|---|
| :HBV.LLA2C.70 | TGA CTG [CG]CG ATT GGT [GA]GA GGC AGG [AC]GG AGG TTA GGC ATA GGA CCC GTG TC |
| :HBV.LLA2C.69 | CTT G[AT][CT] GGG [GA]TT GAA GTC CCA ATC TGG ATT TTA GGC ATA GGA CCC GTG TC |

:HBV.LLA2C.68    GTT GCG TCA GCA AAC ACT TGG CA[CG]
AGA CC[AT] TTA GGC ATA GGA CCC GTG TC

:HBV.LLA2C.67    TAA GTT GGC GAG AAA GT[GA] AAA GCC
TG[TC] TT[AC] TTA GGC ATA GGA CCC GTG
TC

:HBV.LLA2C.66    GCA GCA AA[GA] CCC AAA AGA CCC ACA
A[TG][TA] C[TG][TC] TTA GGC ATA GGA
CCC GTG TC

:HBV.LLA2C.65    ATG TAT ACC CA[GA] AGA CA[AG] AAG AAA
ATT GGT TTA GGC ATA GGA CCC GTG TC

:HBV.LLA2C.59    TAG AGG ACA AAC GGG CAA CAT ACC TTG
[AG]TA TTA GGC ATA GGA CCC GTG TC

:HBV.LLA2C.58    GAT GAG GCA TAG CAG CAG GAT GAA GAG
GAA TTA GGC ATA GGA CCC GTG TC

:HBV.LLA2C.57    GAT AAA ACG CCG CAG ACA CAT CCA GCG
ATA TTA GGC ATA GGA CCC GTG TC

:HBV.LLA2C.56    GGA CAA [AG]TT GGA GGA CA[GA] GAG GTT
GGT GAG TTA GGC ATA GGA CCC GTG TC

:HBV.LLA2C.55    TTG GAG GTT GGG GAC TGC GAA TTT TGG
CCA TTA GGC ATA GGA CCC GTG TC

:HBV.LLA2C.54    CCA CCA CGA GTC TAG ACT CTG [CT]GG
TAT TGT TTA GGC ATA GGA CCC GTG TC

:HBV.LLA2C.53    GAT TCT TGT CAA CAA GAA AAA CCC CGC
CTG TTA GGC ATA GGA CCC GTG TC

```
:HBV.LLA2C.52      CAC GAG [CA]AG GGG TCC TAG GAA TCC
                   TGA TGT TTA GGC ATA GGA CCC GTG TC


:HBV.LLA2C.51      CAG GGT TTA CTG TTC C[TG]G AAC TGG
                   AGC CAC TTA GGC ATA GGA CCC GTG TC
```

A set of single-stranded analyte capture probes, each having a varying 30 base-long portion complementary to a specific sequence of the constant ds region of the HBV genome and a constant 20 base long 3'-portion complementary to the oligonucleotide bound to a microtiter dish as described in Example 2 is synthesized as described in Example 2. The sequences of these probes are presented in Table 2 below.

## TABLE 2
## Analyte Capture Probes for HBV

| Probe Designation | Sequence |
|---|---|
| :HBV.XT1.64 | CTT GGC CCC CAA TAC CAC ATC ATC CAT ATA CTT CTT TGG AGA AAG TGG TG |
| :HBV.XT1.63 | GAA AGC CAA ACA GTG GGG GAA AGC CCT ACG CTT CTT TGG AGA AAG TGG TG |
| :HBV.XT1.62 | CAC TGA ACA AAT GGC ACT AGT AAA CTG AGC CTT CTT TGG AGA AAG TGG TG |
| :HBV.XT1.61 | GAG AAA CGG [AG]CT GAG GCC C[AC]C TCC CAT AGG CTT CTT TGG AGA AAG TGG TG |
| :HBV.XT1.60 | [GC]CG AAA GCC CAG GA[CT] GAT GGG ATG GGA ATA CTT CTT TGG AGA AAG TGG TG |

All other methods and reagents are the same as Example 2.

Example 6

Hybridization Assay for TEM-1 beta-Lactamase DNA in N. gonorrhoeae Using the Microtiter Dish Assay Procedure and T7 RNA polymerase

Molecular analyses have revealed that the penicillin resistance observed in N. gonorrhoeae is mostly due to the presence of a TEM-1 beta-Lactamase gene in a nonconjugative plasmid of 3-7 M. daltons. (This plasmid is homologous to those found in H. ducreyi, H. parainfluenzae, and occasionally H. influenzae.) A hybridization assay is thus developed to detect TEM-1 DNA in N. gonorrhoeae (or the other aforementioned bacteria carrying homologous plasmids) for the purpose of determining penicillin resistance.

The 7.3 Kb N. gonorrhoeae plasmid carrying the TEM-1 gene has been obtained and a segment containing 80% of the TEM-1 gene was sequenced as described in commonly owned EPA Publication No. 0317077. Analyte capture and template linker probes are synthesized and purified as described in Example 2, supra. The 5'-portion of the template linker probes are complementary to sequences of the coding region of the gene; whereas the 5'-portions of the analyte capture probes are complementary to adjoining sequences of the plasmid. Alternatively, probes are also prepared in which the 5'-portions of both sets are directed to the TEM-1 gene.

In all other respects the hybridization assay procedure and reagents are the same as described in Example 2.

This TEM-1 assay is a powerful clinical tool that will enable medical personnel to identify penicillin-resistant infection and optimize a treatment regime by choosing an appropriate antibiotic therapy.

Example 7

Hybridization Assay for Chlamydia trachomatis DNA Using the Microtiter Dish Assay Procedure and T7 Polymerase

Template linker and analyte capture probes are prepared using the same strategy as described in Example 2 and designed to hybridize to the Chlamydia pCHL2 plasmid described by Palmer and Falkow (Plasmid (1986) 16: 52-62). Each probe of the set is a 50 mer in which the first 30 5'-residues are complementary to pCHL2 sequences and the remaining 3'-residues are the system-specific analyte capture and template linker sequences described in Example 2. The pCHL2 sequences used to design these probes are disclosed in commonly owned EPA Publication No. 0317077.

In all other respects the hybridization assay procedure and reagents are the same as describe in Example 2.

Example 8

Hybridization Assay for tet M Determinant in N. gonorrhoeae

N. gonorrhoeae strains resistant to high levels of tetracycline, exhibiting minimum inhibitory concentration values above 16 g/ml, have been found to have acquired the tet M determinant in a 24.5 Md conjugative plasmid (Cannon, J.G., et al., Annual Review of Microbiology (1984) 38:111; Morse, S.A., et al., Antimicrob. Agents Chemother. (1986) 30:664). A hybridization assay is thus developed to detect tet M DNA in N. gonorrhoeae (or the other aforementioned bacteria carrying homologous plasmids) for the purpose of determining tetracycline resistance.

Ten μl of tetracycline resistant N. gonorrhoeae (TRNG) cells suspended in either GC broth or skimmed milk are mixed with 12.5 μl of lysis solution (2mg/ml proteinase K in 10mM Tris-HCl, 150 mM NaCl, 10 mM EDTA, 1% SDS, pH 8.0) in a clear Immulon™ II well (Dynatech), and incubated at 65°C for 20 min.

Analyte capture and template linker probes are synthesized and purified as described in Example 2, supra, except they are designed to hybridize to the tet M structural gene. The sequences of the probes are based on the tet M gene sequence from the streptococcal conjugative shuttle transposon Tn 1545 described in Martin, P., et al., Nuc. Acids Res. (1986) 14:7047.

In all other respects the hybridization assay-procedure and reagents are the same as described in Example 2.

Example 9

Comparison of Template Probes with Various Numbers of Base Pairs Between the A and B Domain in Assays for the Presence of Human Immunodeficiency Virus (HIV) DNA in Human Plasma.

Various template probes were prepared, each having the same functional domains, A, B, and C, but with different numbers of base pairs separating the A domain and the T7 promoter.

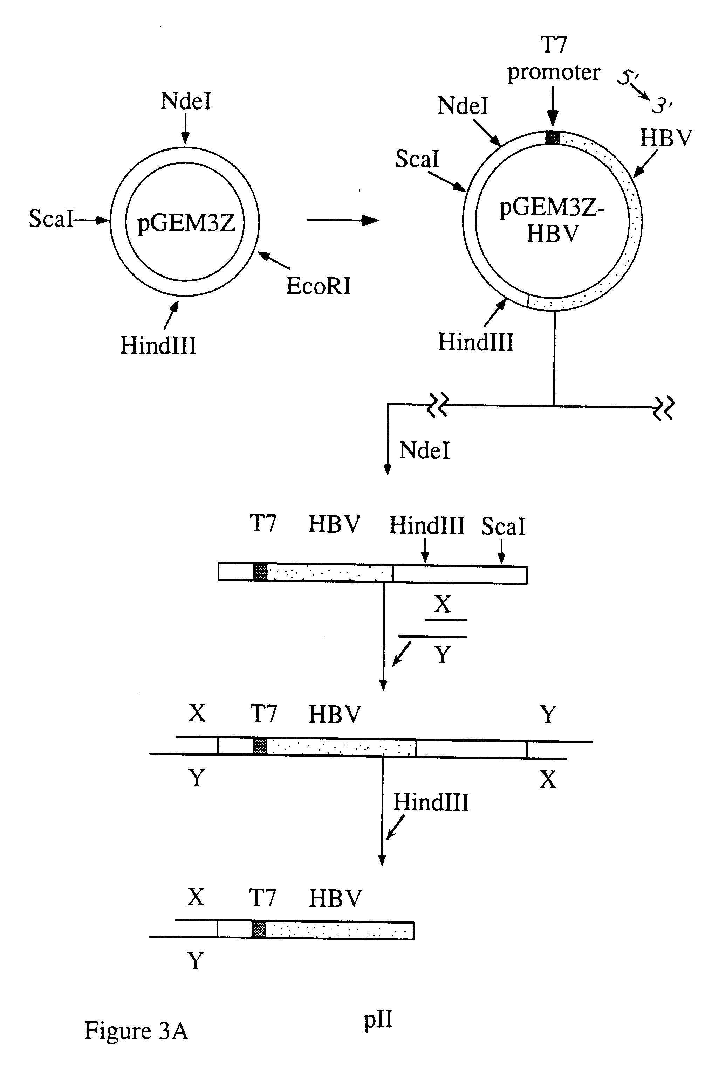

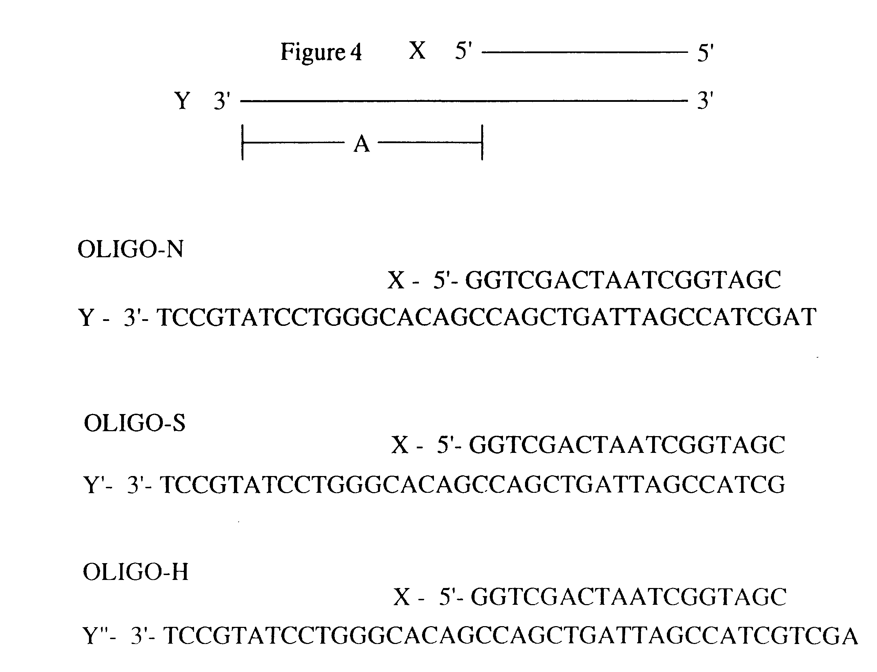

The general strategy was to prepare DNA containing the T7 polymerase promoter operably linked to the 5' end of the Hepatitis B viral genome (HBV) - about 3.4 Kb in length. Thus, the HBV genome acts as domain C and functions as the template for subsequent transcription while the resulting HBV-specific RNA functions as reporter transcripts. This DNA fragment was cloned in plasmid pGEM3Z (commercially available from Promega, Inc.- see Figure 3A). Next, a partially single stranded oligomer, corresponding to the A domain of the template probes, and a short (18 nucleotide) double stranded spacer region with a cohesive end was also prepared (Figure 4). The oligomer was then ligated to the promoter/HBV DNA fragment (B/C domain) which had been isolated from the plasmid by restriction endonuclease digestion and subsequent purification. The size of the fragment varied depending on the restriction endonuclease used to linearize the plasmid (Figures 3A-C).

### A. pII template probe

#### 1. T7 promoter (domain B) and HBV (domain C)

A DNA segment comprised of the T7 consensus sequence, oriented directly 5' to linearized HBV genomic DNA, was inserted in the EcoRI site of pGEM3Z (pGEM3Z-HBV). After cloning, the plasmid was isolated and relinearized by digestion with Nde I (Figure 3A).

#### 2. The Oligomer

A partially double stranded oligomer was synthesized which comprised domain A and a short double stranded domain terminating in a cohesive end complementary to the cohesive end created by Nde I digestion. This oligomer is designated oligo N. Oligo N is comprised of two DNA strands. The sequence of strand X is 5'-GGT CGA CTA ATC GGT AGC-3'. The sequence of strand Y is 3'-TCC GTA TCC TGG GCA CAG CCA GCT GAT TAG CCA TCG AT-5'. Strands X and Y were annealed creating a single stranded domain A at the 3'-end of strand Y and an AT cohesive end at the 5'-end of strand Y.

#### 3. Ligation

Oligo N and Nde I linearized pGEM3Z-HBV were ligated, creating a oligonucleotide with oligo N at both ends of the molecule (Figure 3A). The molecule was trimmed with HinD III thereby creating the template probe designated pII (Figure 3A). The distance between the T7 promoter and domain A is 200 base pairs.

### B. pII$_L$ template probe

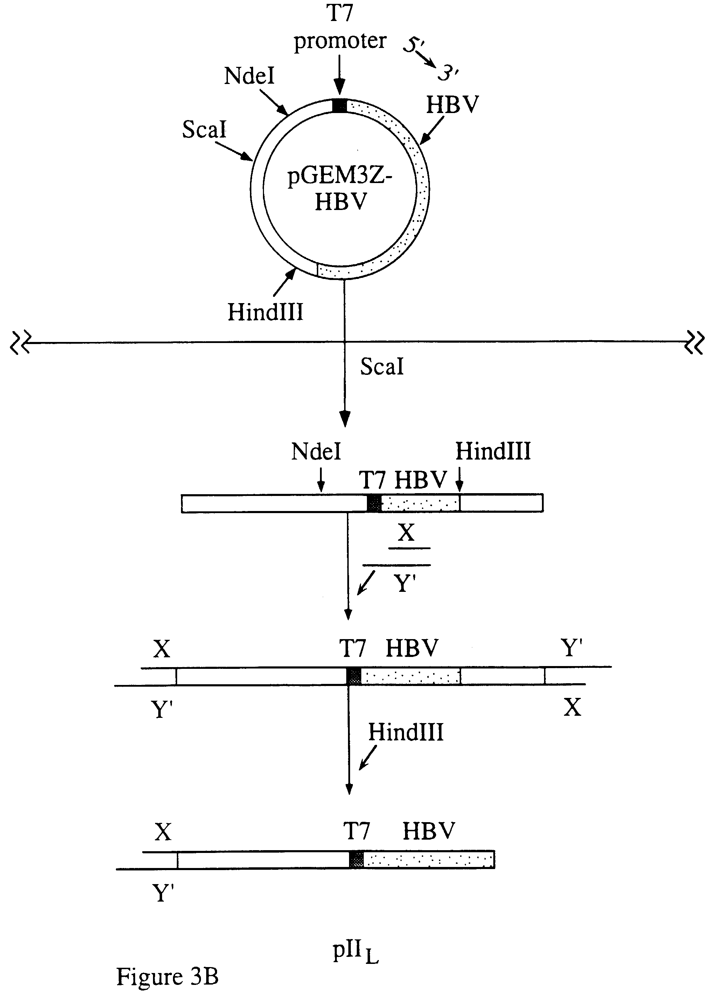

pII$_L$ template probe was synthesized as described in A, _supra_, except that pGEM3Z-HBV was linearized with Sca I, thereby creating a longer spacer region between the T7 promoter of domain B and domain A (Figure 3B). In this case, the oligomer is designated oligo S and consists of strands X and Y'. Oligo S differs from oligo N in that there is no cohesive end. Strand X is the same as strand X in oligo N, but strand Y' lacks the 5'-TA. Strands X and Y' were annealed creating a single stranded domain A at the 3'-end of strand Y' and a blunt end at the 5'-end of strand Y The linearized plasmid and oligo S were blunt-end ligated. The distance between the T7 promoter and domain A is 900 base pairs.

### C. pII$_R$ template probe

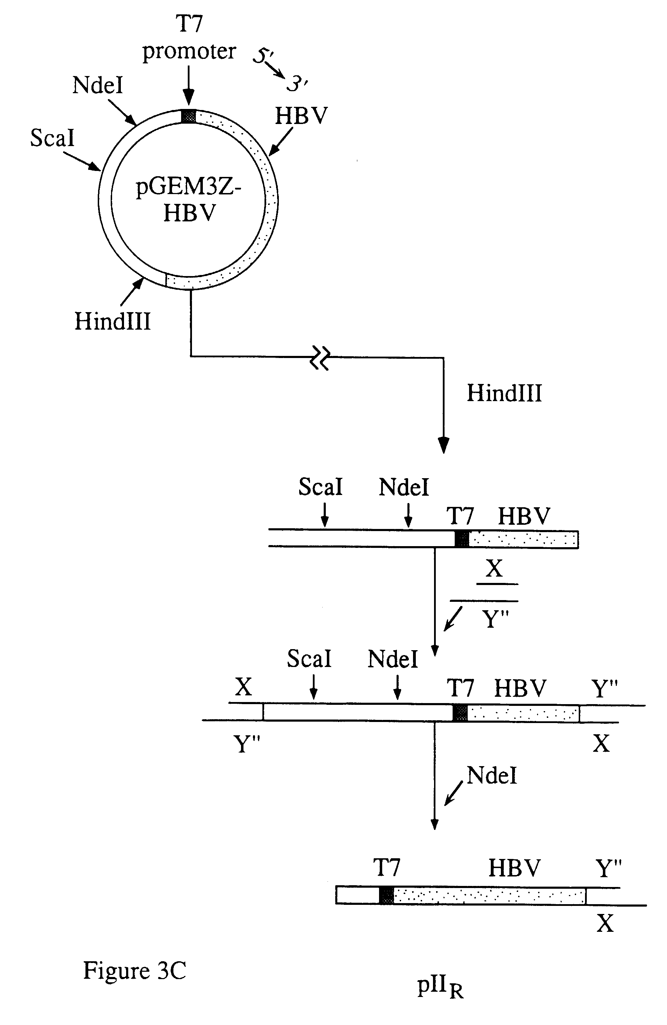

pII$_R$ template probe was synthesized as described in A, _supra_, except that pGEM3Z-HBV was linearized with Hind III (Figure 3C). The oligomer is designated oligo H and consists of strands X and Y". Oligo H differs from oligo N in that the cohesive end is complementary to the cohesive end generated by Hind III digestion. Strand X is the same as strand X in oligo N, but strand Y" has the sequence 3'-TCC GTA TCC TGG GCA CAG CCA GCT GAT TAG CCA TCG TCGA-5'. Strands X and Y" were annealed creating a single stranded domain A at the 3'-end of strand Y" and an TCGA cohesive end at the 5'-end of strand Y". Oligo H and Hind III-linearized pGEM3Z-HBV were ligated, creating a oligonucleotide with oligo H at both ends of the molecule (Figure 3C). The molecule was trimmed with Nde I rather than Hind III, thereby creating the template probe designated pII$_R$. This probe differs from pII and pII$_L$ in that the sequence of domains is B-C-A. The distance between T7 promoter and domain A is 3200 base pairs.

### D. HIV-specific capture and linker probes.

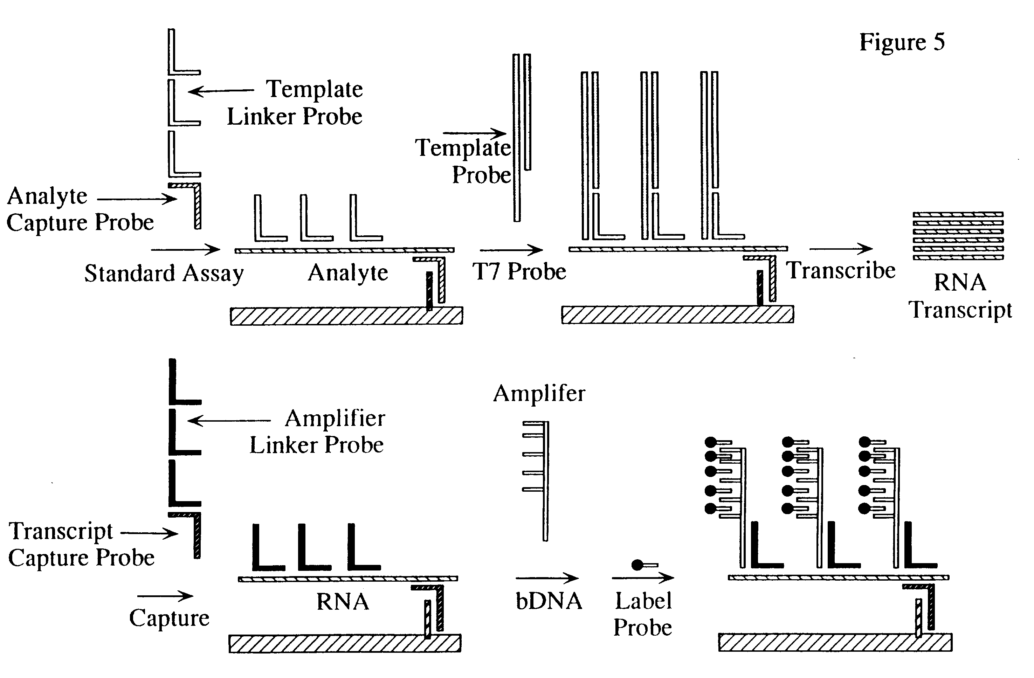

The assays described below (see Figure 5) are similar to the assay described in Example 2 above. Since HIV is the analyte it was necessary to create analyte capture probes and template linker probes with subdomains homologous to the HIV genome. The sequences of these novel linker probes are provided below. The 5'-portion of each probe is complementary to a portion of the HIV genome while the 3'-portion is complementary to an immobilized oligonuleotide capture sequence (in the case of capture probes) or to the A domain of the T7 template probe (in the case of the template linker probes).

## TABLE 3
### Analyte Capture Probes

| Probe Designation | Sequence |
| --- | --- |
| :HIV.96.1.XT1 | TTC TAC TAC TTT [TC]AC CCA TGC [AG]TT TAA AGC TTC TTT GGA GAA AGT GGTG |
| :HIV.96.2.XT1 | TTC TAT TAC TTT [TC]AC CCA TGC [AG]TT CAA AGC TTC TTT GGA GAA AGT GGT G |
| :HIV.97.XT1 | TGC TTG ATG TCC CCC CAC TGT GTT TAG CAT CTT CTT TGG AGA AAG TGG TG |
| :HIV.97.2.XT1 | TGC CTG GTG TCC TCC AAC TAT GTT CAG CAT CTT CTT TGG AGA AAG TGG TG |

:HIV.53.XT1   AGG TGA TAT GGC [CT]TG ATG TA[CT] CAT
TTG CCC CTT CTT TGG AGA AAG TGG TG

:HIV.54.XT1   CAT GGG TAT [TC]AC TTC TGG GCT [GA]AA
[AG]GC CTT CTT CTT TGG AGA AAG TGG TG

:HIV.55.XT1   TTG [TC]GG GGT GGC [TC]CC [TC]TC TGA
TAA TGC TGA CTT CTT TGG AGA AAG TGG
TG

:HIV.68.1.XT1  AAT TTT T[GA]A AAT TTT [TC]CC TTC CTT
TTC CAT CTT CTT TGG AGA AAG TGG TG

:HIV.68.2.XT1  AAC TCT T[GA]A AAT TTT [TC]CC TTC CTT
TTC CAT CTT CTT TGG AGA AAG TGG TG

:HIV.99.XT1   TTA CTG GTA CAG T[TC]T CAA TAG G[AG]C
TAA T[GT]G CTT CTT TGG AGA AAG TGG TG

:HIV.100.XT1  TAA C[TC][TC] TTG GGC CAT CCA T[TC]C
CTG GCT TTC TTC TTT GGA GAA AGT GGT G

:HIV.101.XT1  CTT TTA TTT TTT CTT CTG TCA ATG GCC
ATC TTC TTT GGA GAA AGT GGT G

:HIV.102.XT1  AAA TAC TGG AGT ATT GTA TGG ATT
[CT]TC AGC TTC TTT GGA GAA AGT GGT G

## TABLE 4
### Template Linker Probes

| Probe Designation | Sequence |
|---|---|
| :HIV.51.LLA2C | TCC [CG]CC GCT TAA TAC [TC]GA CGC TCT CGC ACC TTA GGC ATA GGA CCC GTG TC |

```
:HIV.52.LLA2C        TTA [TA]AT AAT GAT [CT]TA AGT TCT TCT
                     GAT CCT TTA GGC ATA GGA CCC GTG TC

:HIV.56.LLA2C        ACT TCC [CT]CT TGG TTC TCT CAT [CT]TG
                     [GA]CC TGG TTA GGC ATA GGA CCC GT GTC

:HIV.57.LLA2C        TTC [CT]TG AAG GGT ACT AGT [AG]GT TCC
                     TGC TAT TTA GGC ATA GGA CCC GTG TC

:HIV.58.1.LLA2C      GAT AGG TGG ATT A[CT][TG] TGT CAT CCA
                     T[GC]C TAT TTA GGC ATA GGA CCC GTG TC

:HIV.58.2.LLA2C      GAT AGG TGG GTT G[TC][TG] TGT CAT CCA
                     T[GC]C TAT TTA GGC ATA GGA CCC GTG TC

:HIV.59.1.LLA2C      ATT ATC CA[TC] CTT TTA TA[GA] ATT TCT
                     CCT ACT TTA GGC ATA GGA CCC GTG TC

:HIV.59.2.LLA2C      ATT ATC CA[TC] CTT TTA TA[GA] ATG TCT
                     CCC ACT TTA GGC ATA GGA CCC GTG TC

:HIV.60.LLA2C        CTA TAC AT[TC] CTT ACT ATT TTA TTT
                     AAT CCC TTA GGC ATA GGA CCC GTG TC

:HIV.62.LLA2C        TT[CT] GCA TTT TGG ACC A[AG][CG] AAG
                     GTT TCT GTC TTA GGC ATA GGA CCC GTG
                     TC

:HIV.63.LLA2C        CTC CCT G[AG]C ATG CTG TCA TCA TTT
                     CTT CTA TTA GGC ATA GGA CCC GTG TC

:HIV.64.1.LLA2C      TTC A[TG]T TGG TGT CCT TCC TT[TC] CCA
                     CAT TTC TTA GGC ATA GGA CCC GTG TC
```

```
:HIV.64.2.LLA2C      TTC A[TG]T TGG TGT CCT TCC CT[TC] CCA
                     CAT CTC TTA GGC ATA GGA CCC GTG TC


:HIV.65.LLA2C        GCC A[GA]A T[CT]T TCC CTA AAA AAT TAG
                     CCT GTC TTA GGC ATA GGA CCC GTG TC


:HIV.98.LLA2C        [AG]TC CCA [TG]TC TGC AGC TTC CTC ATT
                     GAT [GA]GT TTA GGC ATA GGA CCC GTG TC


:HIV.66.LLA2C        ATC ATT TTT GGT TTC CAT [CT]TT C[CT]T
                     GGC AAA TTA GGC ATA GGA CCC GTG TC


:HIV.67.LLA2C        TGT C[TC]T ACT TTG ATA AAA CCT CCA
                     ATT CCC TTA GGC ATA GGA CCC GTG TC


:HIV.70.LLA2C        TCT CCA [TC]TT [AG]GT [AG]CT GTC TTT
                     TTT CTT TAT TTA GGC ATA GGA CCC GTG
                     TC


:HIV.71.LLA2C        GTA CTG ATA TC[TC] A[AC]T CCC TGG TGT
                     [CT]TC ATT TTA GGC ATA GGA CCC GTG TC


:HIV.72.LLA2C        GGT GAT CCT TTC CAT CCC TGT GG[ACT]
                     AGC ACA TTA GGC ATA GGA CCC GTG TC


:HIV.73.LLA2C        TAA GAT TTT TGT CAT GCT AC[TA] [TC]TG
                     GAA TAT TTA GGC ATA GGA CCC GTG TC


:HIV.69.LLA2C        AGA [TC]CC TAC ATA CAA ATC ATC CAT
                     GTA TTG TTA GGC ATA GGA CCC GTG TC


:HIV.74.LLA2C        TAT TTT TG[TC] TCT ATG [CT]TG [CT]CC
                     TAT TTC TAA TTA GGC ATA GGA CCC GTG
                     TC
```

:HIV.75.LLA2C

ATG [TC]TT TTT [GA]TC TGG TGT GGT
AA[GA] TCC CCA TTA GGC ATA GGA CCC
GTG TC

:HIV.76.LLA2C

ATA [AC]CC CAT CCA AAG [GA]AA TGG
[AG]GG TTC TTT TTA GGC ATA GGA CCC
GTG GTC

:HIV.77.LLA2C

TA[CT] TAA GTC TTT TGA TGG GTC ATA
ATA [TC]AC TTA GGC ATA GGA CCC GTG TC

:HIV.78.1.LLA2C

TGT TTT CAG ATT TTT AAA TGG [CT]TC
TTG ATA TTA GGC ATA GGA CCC GTG TC

:HIV.78.2.LLA2C

TGT TTT CAG ATT TTT ATA TTG [CT]TC
TTG GTA TTA GGC ATA GGA CCC GTG TC

:HIV.79.LLA2C

G[TC]T AA[TC] TGT TT[TC] ACA TCA TTA
GTG TGG GCA TTA GGC ATA GGA CCC GTG
TC

:HIV.80.LLA2C

GGA [GA]T[CT] TTT CCC CAT AT[TC] ACT
ATG CTT TCT TTA GGC ATA GGA CCC GTG
TC

:HIV.81.LLA2C

CCC CAT CTA CAT AGA A[GAC]G TTT CTG
C[TA]C CTA TTA GGC ATA GGA CCC GTG TC

:HIV.82.LLA2C

TGC TTG TAA [CT]TC AGT [TC]TT CTG ATT
TGT TGT TTA GGC ATA GGA CCC GTG TC

:HIV.83.LLA2C

ATC TGG TTG TGC TTG AAT [GA]AT [TC]CC
[TC]AA TGC ATT AGG CAT AGG ACC CGT
GTC

EP 0 510 085 B1

:HIV.84.LLA2C          ATC TAC TTG TTC ATT TCC TCC AAT
                       [TC]CC TTT TTA GGC ATA GGA CCC GTG TC

:HIV.85.LLA2C          TAG CCA TTG CTC TCC AAT T[AG][CT] TGT
                       GAT ATT TTA GGC ATA GGA CCC GTG TC

:HIV.86.LLA2C          GAC ATT TAT CAC AGC T[GA]G CTA CTA
                       TTT C[TC]T TTA GGC ATA GGA CCC GTG TC

:HIV.87.LLA2C          TAT [AG]TA [TG]CC ACT GGC TAC ATG
                       [AG]AC TGC TAC TTA GGC ATA GGA CCC
                       GTG TC

:HIV.88.LLA2C          TTT TAC TGG CCA TCT TCC TGC TAA TTT
                       TAT TAG GCA TAG GAC CCG TGT C

:HIV.89.LLA2C          TAC TCC TTG ACT TTG GGG [AG]TT GTA
                       GGG AAT TTA GGC ATA GGA CCC GTG TC

:HIV.90.LLA2C          TCT [TC]TC CCC TGC ACT GTA [CT]CC CCC
                       AAT CCC TTA GGC ATA GGA CCC GTG TC

:HIV.91.LLA2C          TAG TTT GTA TGT CTG TTG CTA T[TC]A
                       TG[TC] CTA TTA GGC ATA GGA CCC GTG TC

:HIV.92.LLA2C          TTT GAA TTT TTG T[AG]A TTT G[TC]T TTT
                       GTA [AG]TT TTA GGC ATA GGA CCC GTG TC

:HIV.93.LLA2C          TCC AGA G[GTA]A G[CT]T TTG CTG GTC
                       CTT TCC AAA TTA GGC ATA GGA CCC GTG
                       TC

:HIV.94.LLA2C          TAT T[AG]T C[TC]T GTA TTA CTA CTG CCC
                       CTT CAC TTA GGC ATA GGA CCC GTG TC

23

```
:HIV.95.1.LLA2C      TT[GA] CTT TTC TTC TTG GCA CTA CTT
                     TTA T[GA]T TTA GGC ATA GGA CCC GTG TC


:HIV.95.2.LLA2C      TT[GA] CTT TTC TTC TTG GTA CTA CCT
                     TTA T[GA]T TTA GGC ATA GGA CCC GTG TC


:HIV.103.LLA2C       TTT TCT TTT AAA ATT GTG [AG]AT GAA
                     [TC]AC TGC TTA GGC ATA GGA CCC GTG TC
```

D. Comparison of the pII template probes in the assay of HIV in human plasma.

Samples of normal human plasma with varying amounts of a synthetic HIV target sequence were prepared as described in Example 2. 10 µl of plasma were added to 12.5 µl of extraction buffer (10mM Tris pH 8.0, 150 mM NaCl, 10 mM EDTA, 1% SDS 40µg/ml sonicated salmon sperm DNA, and 2mg/ml proteinase K) and incubated in wells of a microtiter dish at 65°C for 30 minutes. The wells were first prepared by binding a single stranded oligonucleotide with a defined sequence to the solid substrate as described in Example 2. 5µl of 1N NaOH with 12.5 fmoles of HIV capture and linker probes/well (described above) were added and the mixture was further incubated at 65°C for 30 minutes. Next 13 µl of MOPS-SSC (0.77 M 3-[N-morpholino]propanesulfonic acid, 1.845 M NaCl, 0.185 M Na Citrate) were added and the mixture further incubated at 65°C for 2 hours.

Wells were then washed 2 times with wash buffer A (0.1 X SSC, 0.1% SDS). Following the washes, 30 fmoles of T7 template probe in 40 µl horse hyb mix (50% horse serum, 0.6 M NaCl, 0.06 M Na Citrate, .1% SDS) were added and the mixture incubated at 55°C for 1 hour. (The horse hybridization mix was prepared as follows: for ten ml - 504 µl water (treated with diethyl pyrocarbonate, DEPC), 336 µl 10% SDS, 60 µl 1 M Tris HCl (pH8), 100µl of 25mg/ml proteinase K, 5 ml horse serum, incubated 65°C for 2 hours, add 1 ml water (DEPC treated) and 2 ml 20X SSC).

The wells were then washed 2 times with wash buffer A and 2 times with wash buffer B (0.1X SSC). Next 40 µl of transcription mix (40 mM Tris-HCl pH 8, 20 mM $MgCl_2$, 80 units T7 polymerase (New England Biolabs), 10mM DTT, 0.15 mg/ml BSA, 1.25 mM each of ATP, UTP, GTP, and CTP, 1600 units/ml RNAsin) were added and the mixture incubated in a 37 degree oven for 1.5 hours.

New wells were again prepared by binding single stranded oligonucleotide with a defined sequence to the solid substrate (described in Example 2). To the new well was added 12.5 µl of extraction buffer (2mg/ml proteinase K), 5µl of proteinase K/SDS treated human serum, 15µl 20X SSC, 5 µl 10% SDS containing 12.5 fmoles of transcript capture and amplifier linker probes, and 12.5 µl of the mixture from the first well which contains the newly formed RNA transcripts. This mixture was incubated 65°C for 2 hours.

Note that, in contrast to the protocol of Example 2, transcript capture and amplifier linker probes are used to sandwich the RNA transcript (Figure 5). These probes serve as bridges between the transcript and the immobilized nucleotide on the one hand, and between the transcript and the amplifier probe (to be added) on the other hand.

The wells are next washed 2 times with wash buffer A. Forty µl of horse hybridization mix containing 100 fmoles of comb-like amplifier probe (as in Example 2) added to each well and incubated at 55°C for 15 minutes. Wells were then washed 2X with wash buffer A. Forty µl of horse hybridization mix containing 100 fmoles of alkaline phosphatase probe is added to each well and incubated at 55°C for 15 minutes. (The horse hybridization mixture is pretreated to remove residual RNAse activity according to the preparation protocol described above except that, after the 65 degree incubation, the solution is cooled and 60µl of 100 mM phenyl-methyl sulfonyl fluoride (PMSF) is added to inactivate the proteinase K, and the mixture is further incubated at 37°C for 1 hour).

The samples were assayed by alkaline phosphatase detection as described in Example 2 above. The wells were washed twice with wash buffer A, twice with wash buffer B. 20µl of dioxetane reagent were added and incubated at 37°C for 30 minutes and the wells were then read in a luminometer.

E. Results

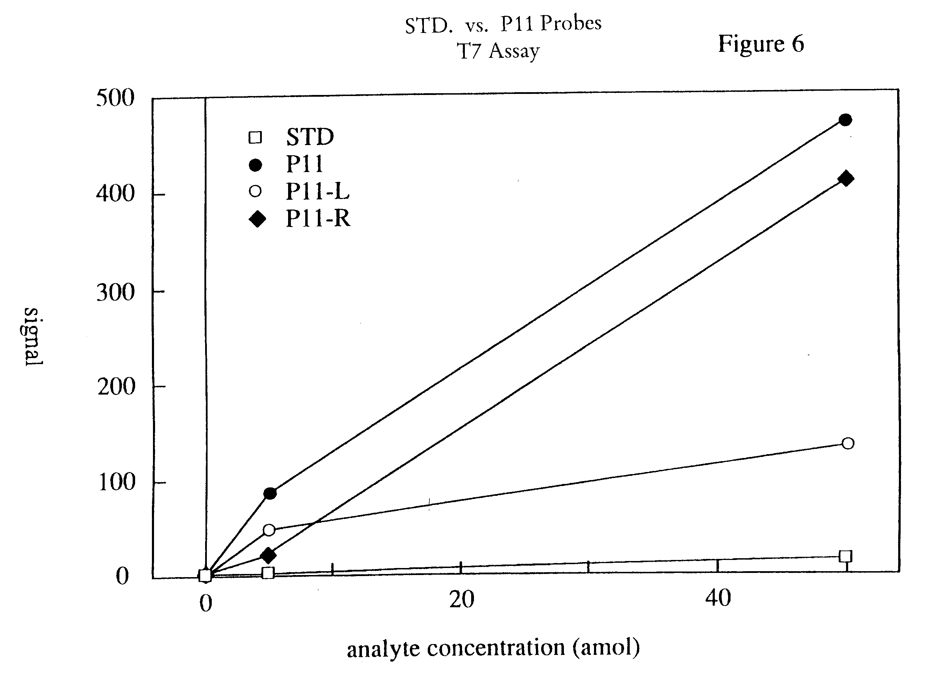

Panels of various amounts of cloned HIV DNA added to samples of human serum were prepared and assayed as described above. Each panel was assayed using one of the three PII T7-template probes described above. The sensitivity and amplification are shown in Figure 6. As a control standard, a panel of HIV DNA was assayed using only the comb-like signal amplification probe assay described in E.P. Pub. 0317077. The use of the T7 template probe provides an approximate 30 fold increase in sensitivity and an approximate 50 fold increase in amplification relative to the non-polymerase standard.

Example 10

Hybridization Assay for Hepatitis C Virus (HCV) DNA Using the Microtiter Dish Assay Procedure and T7 RNA Polymerase

Assays for the presence of HCV-specific DNA and RNA are preformed as described in Example 9. Sets of template linker and analyte capture probes are prepared as described in Example 2 and designed to hybridize to portions of the HCV genome. These probes are disclosed in the commonly owned PCT US90/02853, filed 18 May 1990. Each probe of the set is a 50-mer in which the first 30 residues at the 5'-end are complementary to HCV sequences and the remaining residues are the system -specific analyte capture and template linker sequences described in Example 2.

In the same manner, other pathogenic bacterial, viral and parasitic strains and antibiotic resistance-conferring genes can be screened. It will be appreciated that the invention assay may be adapted to conduct multiple assays for different analytes simultaneously. In one format, by changing the label and the amplifier probe sequences for a new analyte (as well as the analyte specific sequences in the analyte capture and template linker probes) it is possible to detect two different analytes in the same sample on the same solid phase. Alternatively, by synthesizing different oligonucleotides bound to the solid support (Example 2B, supra) for each analyte, and attaching each bound oligonucleotide sequence to different positions on a membrane strip, it is possible to perform several different assays simultaneously with the same label.

**Claims**

**Claims for the following Contracting States : AT, BE, CH, LI, DE, DK, FR, GB, GR, IT, LU, NL, SE**

1. An isolated polydeoxynucleotide construct for use as a signal amplifier in hybridization assays comprising three domains:

   (a) a first domain (A) which is single-stranded and has a nucleotide sequence complementary to a target sequence;
   (b) a second domain (B) which is double-stranded and capable of functioning as a promoter for a DNA-dependent RNA polymerase enzyme activity; and
   (c) a third domain (C) which is either single or double-stranded and adjacent to said second domain, such that said third domain is capable of functioning as a template for the promoter activity of said second domain, wherein the domains of the construct are oriented A-B-C or B-C-A, and further wherein the third domain does not contain target sequence.

2. The polydeoxynucleotide construct of claim 1 in which said promoter is capable of functioning as a promoter for DNA-dependent RNA polymerase derived from the bacteriophage T7.

3. The polydeoxynucleotide construct of claim 1 in which said promoter is capable of functioning as a promoter for DNA-dependent RNA polymerase derived from the bacteriophage T3.

4. The polydeoxynucleotide construct of claim 1 in which said promoter is capable of functioning as a promoter for DNA-dependent RNA polymerase derived from the bacteriophage SP6.

5. The polydeoxynucleotide construct of claim 1 in which said first domain A is at least 10 and no more than 40 nucleotides long.

6. The polydeoxynucleotide construct of claim 1 in which said first domain A is at least 15 and no more than 30 nucleotides long.

7. The polydeoxynucleotide construct of claim 5 in which said second domain B is at least 12 and no more than 40 nucleotides long.

8. The polydeoxynucleotide construct of claim 5 in which said second domain B is at least 17 and no more than 30 nucleotides long.

9. The polydeoxynucleotide construct of claim 7 in which said third domain C is at least 30 and no more than 10000 nucleotides long.

10. The polydeoxynucleotide construct of claim 7 in which said third domain C is at least 40 and no more than 80 nucleotides long.

11. The polydeoxynucleotide construct of claim 7 in which said third domain C is at least 2 Kb and no more than 10 Kb in length.

12. The polydeoxynucleotide construct of claim 7 in which said third domain C is at least 3 Kb and no more than 4 Kb in length.

13. The polydeoxynucleotide construct of claim 7 in which said third domain C is comprised of substantially all of the genome of Hepatitis B virus.

14. The polydeoxynucleotide construct of claim 2 in which the sequence for said second domain B comprises the sequence:

```
5'-TAA TAC GAC TCA CTA TA-3'
3'-ATT ATG CTG AGT GAT AT-5'
```

15. The polydeoxynucleotide construct of claim 2 in which the nucleotide sequence of said second domain B is:

```
5'-CTG GCT TAT CGA AAT TAA TAC GAC TCA CTA TA-3'
3'-GAC CGA ATA GCT TTA ATT ATG CTG AGT GAT AT-5'.
```

16. The polydeoxynucleotide construct of claim 1 in which the 5' residue of the upper strand of the nucleotide sequence for said third domain C, is adjacent to said second domain B, and is a guanosine residue.

17. The polydeoxynucleotide construct of claim 1 in which the sequence for said third domain C is:

```
5'-GGG AGA TGT GGT TGT CGT ACT TAG CGA AAT ACT GTC CGA
GTC G-3'
3'-CCC TCT ACA CCA ACA GCA TGA ATC GCT TTA TGA CAG GCT
CAG C-5'
```

18. The polydeoxynucleotide constructs of claim 17 in which the 3' end of the upper strand of the DNA nucleotide sequence of said second domain B is attached to the 5' end of the upper strand of said nucleotide sequence of domain C.

19. The polydeoxynucleotide construct of claim 1 in which the transcript of said third domain C has two subdomains:

(a) a first subdomain, $c_1$, which is capable of hybridizing to an oligonucleotide capture linker, said capture linker being capable of hybridizing to a polynucleotide immobilized on a solid substrate; and

(b) a second subdomain, $c_2$ which is capable of binding to an oligonucleotide amplifier linker, said amplifier linker being capable of binding to a quantifiable probe.

20. The polydeoxynucleotide construct of claim 1 in which said second and third domains, B and C, are present in multiple repeating units.

21. A method of amplifying a signal used to detect and quantify an oligonucleotide analyte in a hybridization assay comprising:

(i) immobilizing said analyte, directly or indirectly, on a solid substrate; and hybridizing the polydeoxynucleotide construct of claim 1, directly or indirectly to the analyte;

(ii) removing unhybridized polydeoxynucleotide constructs;

(iii) transcribing multiple copies of RNA oligomers which are complementary to the template sequence, c', of said third domain, C, of said polydeoxynucleotide construct via a DNA-dependent RNA polymerase activity; and (iv) detecting the amount of RNA transcripts formed in step (iii).

22. The nucleic acid hybridization assay of claim 21 in which said second domain, B, provides a promoter for the DNA-dependent RNA polymerase of the bacteriophage T7.

23. The nucleic acid hybridization assay of claim 21 in which said second domain, B provides a promoter for the DNA-dependent RNA polymerase of the bacteriophage T3.

24. The nucleic acid hybridization assay of claim 21 in which said second domain, B provides a promoter for the DNA-dependent RNA polymerase of the bacteriophage SP6.

25. The nucleic acid hybridization assay of claim 21 in which said polydeoxynucleotide construct is hybridized directly to a single-stranded analyte.

26. The nucleic acid hybridization assay of claim 21 wherein said polydeoxynucleotide construct is hybridized to an oligonucleotide linker, said linker having a domain which is capable of forming stable hybrids with the analyte.

27. The nucleic acid hybridization assay of claim 21 in which:

(a) said third domain, C, of said polydeoxynucleotide construct is transcribed in the presence of labeled ribonucleotide triphosphates;

(b) the transcripts of said third domain have a first subdomain, $c_1$ complementary to a oligonucleotide capture probe which is immobilized on a solid substrate; and

(c) said transcripts are immobilized and quantified.

28. The nucleic acid hybridization assay of claim 21 in which:

(a) said third domain, C, of said polydeoxynucleotide construct is transcribed in the presence of biotinylated ribonucleotides triphosphates;

(b) said transcript of said third domain has a first subdomain, $c_2$, which is complementary to a labeled oligonucleotide probe; and

(c) said transcripts are immobilized upon an avidinylated solid substrate; and

(d) said transcripts are quantified.

29. The nucleic acid hybridization assay of claim 21 in which:

(a) said third domain, C, of said polydeoxynucleotide construct is transcribed in the presence of both labeled and biotinylated ribonucleotide triphosphates;

(b) said transcripts are immobilized upon an avidinylated solid substrate; and

(c) said transcripts are quantified.

30. The nucleic acid hybridization assay of claim 21 in which:

(a) the transcript of said third domain, C, of said polydeoxynucleotide construct has two subdomains:

(i) a first subdomain, $c_1$, which is complementary to an oligonucleotide capture probe, said probe being immobilized on a solid substrate; and
(ii) a second subdomain, $c_2$, which is complementary to a labeled oligonucleotide probe;

(b) said transcript is hybridized to said capture probe;
(c) said labeled probe is hybridized to said transcripts; and,
(d) said transcripts are quantified.

31. The nucleic acid hybridization assay of claim 21 in which:

(a) the transcript of said third domain, C, of said polydeoxynucleotide construct has two subdomains:

(i) a first subdomain, $c_1$, which is complementary to a transcript capture probe, said transcript capture probe being capable of hybridizing to an oligonucleotide which has been immobilized on a solid substrate; and
(ii) a second subdomain, $c_2$, which is complementary to a linker probe, said linker probe being capable of hybridizing to a labeling oligonucleotide;

(b) said transcript is hybridized to said transcript capture probe and to said linker probe to form a transcript sandwich;
(c) said transcript sandwich is hybridized to an oligonucleotide immobilized on a solid substrate;
(d) said immobilized sandwich is hybridized to a labelling oligonucleotide; and
(e) said transcripts are quantified.

32. The nucleic acid hybridization assay of claim 21 in which:

(a) the transcript of said third domain, C, of said polydeoxynucleotide construct has two subdomains:

(i) a first subdomain, $c_1$, which is complementary to a transcript capture probe, said transcript capture probe being capable of hybridizing to an oligonucleotide which has been immobilized on a solid substrate; and
(ii) a second subdomain, $c_2$, which is complementary to an amplifier linker probe, said linker probe being capable of hybridizing to an amplifier probe;

(b) said transcript is hybridized to said transcript capture probe and to said amplifier linker probe to form a transcript sandwich;
(c) said transcript sandwich is hybridized to an oligonucleotide immobilized on a solid substrate;
(d) said immobilized sandwich is hybridized to an amplifier probe;
(e) said amplifier probe is hybridized to a labeling oligonucleotide; and
(e) said transcripts are quantified.

33. The nucleic acid hybridization assay of claim 32 in which the RNA transcript is transcribed from DNA of Hepatitis B Virus.

34. The nucleic acid hybridization assay of claim 21 in which said second and third domains, B and C, of said polydeoxynucleotide construct are present in multiple repeating units.

35. The method of claim 21 used to detect the presence of <u>N. gonorrhoeae</u> in a biological sample in which the analyte comprises a DNA or RNA segment of <u>N. gonorrhoeae.</u>

36. The method of claim 21 used to detect the presence of Hepatitis B virus in a biological sample in which the analyte comprises a DNA or RNA segment of Hepatitis B virus.

37. The method of claim 21 used to detect the presence of bacteria containing the beta-Lactamase TEM-1 gene, in a biological sample in which the analyte comprises a DNA or RNA segment of the beta-Lactamase TEM-1 gene.

**38.** The method of claim 21 used to detect the presence of <u>Chlamydia</u> in a biological sample in which the analyte comprises a DNA or RNA segment of <u>Chlamydia.</u>

**39.** The method of claim 21 used to detect the presence of bacteria containing the tet M determinant in a biological sample in which the analyte comprises a DNA or RNA segment of the tet M determinant.

**40.** The method of claim 21 used to detect the presence of HIV in a biological sample in which the analyte comprises a DNA or RNA. segment of HIV.

**41.** The method of claim 21 used to detect the presence of HCV in a biological sample in which the analyte comprises DNA or RNA segment of HCV.

**42.** A method of amplifying the signal used to detect and quantify a ligand receptor in an assay comprising the following steps:

(a) immobilizing said ligand receptor directly or indirectly on a solid phase;
(b) binding to the ligand receptor a ligand specific for said receptor, said ligand being coupled to an oligonucleotide complementary to the first domain of the construct of claim 1;
(c) removing unbound ligand;
(d) hybridizing the construct of claim 1 to the olignucleotide;
(e) removing unhybridized constructs;
(f) transcribing multiple copies of RNA oligomers which are complementary to the template sequence, c', of the third domain, c, of said polydeoxynucleotide construct via a DNA-dependent RNA polymerase activity; and
(g) quantifying the RNA transcripts.

**43.** The hybridization assay of claim 42 in which the ligand receptor is an antigen and the ligand is an antibody which immunologically reacts with the antigen.

**Claims for the following Contracting State : ES**

**1.** A method of amplifying a signal used to detect and quantify an oligonucleotide analyte in a hybridization assay comprising:

(i) immobilizing said analyte directly or indirectly on a solid substrate and hybridizing directly or indirectly to said analyte a polydeoxynucleotide construct comprising three domains:

(a) a first domain (A) which is single-stranded and has a nucleotide sequence complementary to a target sequence;
(b) a second domain (B) which is double-stranded and capable of functioning as a promoter for a DNA-dependent RNA polymerase enzyme activity; and
(c) a third domain (C) which is either single- or double-stranded and adjacent to said second domain, such that said third domain is capable of functioning as a template for the promoter activity of said second domain,

wherein the domains of the construct are oriented A-B-C or B-C-A, and further wherein the third domain does not contain target sequence.
(ii) removing unhybridized polydeoxynucleotide constructs;
(iii) transcribing multiple copies of RNA oligomers which are complementary to the template sequence, c', of said third domain, C, of said polydeoxynucleotide construct via a DNA-dependent RNA polymerase activity; and
(iv) detecting the amount of RNA transcripts formed in step (iii).

**2.** The nucleic acid hybridisation assay of claim 1 in which said second domain, B, provides a promoter for the DNA-dependent RNA polymerase of the bacteriophage T7.

**3.** The nucleic acid hybridization assay of claim 1 in which said second domain, B, provides a promoter for the DNA-dependent RNA polymerase of the bacteriophage T3.

**4.** The nucleic acid hybridization assay of claim 1 in which said second domain, B, provides a promoter for the DNA-dependent RNA polymerase of the bacteriophage SP6.

**5.** The nucleic acid hybridization assay of any one of claims 1 to 4 in which said first domain A of said polydeoxynucleotide construct is at least 10 and no more than 40 nucleotides long.

**6.** The nucleic acid hybridization assay of claim 5 in which said first domain A is at least 15 and no more than 30 nucleotides long.

**7.** The nucleic acid hybridization assay of claim 5 in which said second domain B of said polydeoxynucleotide construct is at least 12 and no more than 40 nucleotides long.

**8.** The nucleic acid hybridization assay of claim 7 in which said second domain B is at least 17 and no more than 30 nucleotides long.

**9.** The nucleic acid hybridization assay of claim 7 in which said third domain C of said polydeoxynucleotide construct is at least 30 and no more than 10000 nucleotides long.

**10.** The nucleic acid hybridization assay of claim 7 in which said third domain C of said polydeoxynucleotide construct is at least 40 and no more than 80 nucleotides long.

**11.** The nucleic acid hybridization assay of claim 7 in which said third domain C of said polynucleotide construct is at least 2 Kb and no more than 10 Kb in length.

**12.** The nucleic acid hybridization assay of claim 7 in which said third domain C of said polynucleotide construct is at least 3 Kb and no more than 4 Kb in length.

**13.** The nucleic acid hybridization assay of claim 7 in which said third domain C of said polynucleotide construct is comprised of substantially all of the genome of Hepatitis B virus.

**14.** The nucleic acid hybridization assay of claim 2 in which the sequence for said second domain B comprises the sequence:

```
5'-TAA TAC GAC TCA CTA TA-3'
3'-ATT ATG CTG AGT GAT AT-5'
```

**15.** The nucleic acid hybridization assay of claim 2 in which the nucleotide sequence of said second domain B is:

```
5'-CTG GCT TAT CGA AAT TAA TAC GAC TCA CTA TA-3'
3'-GAC CGA ATA GCT TTA ATT ATG CTG AGT GAT AT-5'
```

**16.** The nucleic acid hybridization assay of claim 1 in which the 5' residue of the upper strand of the nucleotide sequence for said third domain C, is adjacent to said second domain B, and is a guanosine residue.

**17.** The nucleic acid hybridization assay of claim 1 in which the sequence for said third domain C is:

```
5'-GGG AGA TGT GGT TGT CGT ACT TAG CGA AAT ACT GTC CGA
GTC G-3'
3'-CCC TCT ACA CCA ACA GCA TGA ATC GCT TTA TGA CAG GCT
CAG C-5'
```

18. The nucleic acid hybridization assay of claim 17 in which the 3' end of the upper strand of the DNA nucleotide sequence of said second domain B is attached to the 5' end of the upper strand of said nucleotide sequence of domain C.

19. The nucleic acid hybridization assay of claim 1 in which the transcript of said third domain C has two subdomains:

   (a) a first subdomain, $c_1$, which is capable of hybridizing to an oligonucleotide capture linker, said capture linker being capable of hybridizing to a polynucleotide immobilized on a solid substrate; and
   (b) a second subdomain, $c_2$, which is capable of binding to an oligonucleotide amplifier linker, said amplifier linker being capable of binding to a quantifiable probe.

20. The nucleic acid hybridization assay of claim 1 in which said second and third domains, B and C, of said polydeoxynucleotide construct are present in multiple repeating units.

21. The nucleic acid hybridization assay of claim 1 in which said polydeoxynucleotide construct is hybridized directly to a single-stranded analyte.

22. The nucleic acid hybridization assay of claim 1 wherein said polydeoxynucleotide construct is hybridized to an oligonucleotide linker, said linker having a domain which is capable of forming stable hybrids with the analyte.

23. The nucleic acid hybridization assay of claim 1 in which:

   (a) said third domain, C, of said polydeoxynucleotide construct is transcribed in the presence of labelled ribonucleotide triphosphates;
   (b) the transcripts of said third domain have a first subdomain, $c_1$, complementary to a oligonucleotide capture probe which is immobilized on a solid substrate; and
   (c) said transcripts are immobilized and quantified.

24. The nucleic acid hybridization assay of claim 1 in which:

   (a) said third domain, C, of said polydeoxynucleotide construct is transcribed in the presence of biotinylated ribonucleotides triphosphates;
   (b) said transcript of said third domain has a first subdomain, $c_2$, which is complementary to a labelled oligonucleotide probe; and
   (c) said transcripts are immobilized upon an avidinylated solid substrate; and
   (d) said transcripts are quantified.

25. The nucleic acid hybridization assay of claim 1 in which:

   (a) said third domain, C, of said polydeoxynucleotide construct is transcribed in the presence of both labelled and biotinylated ribonucleotide triphosphates;
   (b) said transcripts are immobilized upon an avidinylated solid substrate; and
   (c) said transcripts are quantified.

26. The nucleic acid hybridization assay of claim 1 in which:

   (a) the transcript of said third domain, C, of said polydeoxynucletoide construct has two subdomains:

      (i) a first subdomain, $c_1$, which is complementary to an oligonucleotide capture probe, said probe being immobilized on a solid substrate; and

(ii) a second subdomain, $c_2$, which is complementary to a labelled olignucleotide probe;

(b) said transcript is hybridized to said capture probe;
(c) said labelled probe is hybridized to said transcripts; and,
(d) said transcripts are quantified.

**27.** The nucleic acid hybridization assay of claim 1 in which:

(a) the transcript of said third domain, C, of said polydeoxynucleotide construct has two subdomains:

(i) a first subdomain, $c_1$, which is complementary to a transcript capture probe, said transcript capture probe being capable of hybridizing to an oligonucleotide which has been immobilized on a solid substrate; and
(ii) a second subdomain, $c_2$, which is complementary to a linker probe, said linker probe being capable of hybridizing to a labelling oligonucleotide;

(b) said transcript is hybridized to said transcript capture probe and to said linker probe to form a transcript sandwich;
(c) said transcript sandwich is hybridized to an oligonucleotide immobilized on a solid substrate;
(d) said immobilized sandwich is hybridized to a labelling oligonucleotide; and
(e) said transcripts are quantified.

**28.** The nucleic acid hybridization assay of claim 1 in which:

(a) the transcript of said third domain, C, of said polydeoxynucleotide construct has two subdomains:

(i) a first subdomain, $c_1$, which is complementary to a transcript capture probe being capable of hybridizing to an oligonucleotide which has been immobilized on a solid substrate; and
(ii) a second subdmain, $c_2$, which is complementary to an amplifier linker probe, said linker probe , said linker probe being capable of hybridizing to an amplifier probe;

(b) said transcript is hybridized to said transcript capture probe and to said amplifier linker probe to form a transcript sandwich;
(c) said transcript sandwich is hybridized to an oligonucleotide immobilized on a solid substrate;
(d) said immobilized sandwich is hybridized to an amplifier probe;
(e) said amplifier probe is hybridized to a labelling oligonucleotide; and
(f) said transcripts are quantified.

**29.** The nucleic acid hybridization assay of claim 28 in which the RNA transcript is transcribed from DNA of Hepatitis B virus.

**30.** The method of claim 1 used to detect the presence of N. gonorrhoeae in a biological sample in which the analyte comprises a DNA or RNA segment of N. gonorrhoeae.

**31.** The method of claim 1 used to detect the presence of Hepatitis B virus in a biological sample in which the analyte comprises a DNA or RNA segment of Hepatitis B virus.

**32.** The method of claim 1 used to detect the presence of bacteria containing the beta-Lactamase TEM-1 gene in a biological sample in which the analyte comprises a DNA or RNA segment of the beta-Lactamase TEM-1 gene.

**33.** The method of claim 1 used to detect the presence of Chlamydia in a biological sample in which the analyte comprises a DNA or RNA segment of Chlamydia.

**34.** The method of claim 1 used to detect the presence of bacteria containing the tet M determinant in a biological sample in which the analyte comprises a DNA or RNA segment of the tet M determinant.

**35.** The method of claim 1 used to detect the presence of HIV in a biological sample in which the analyte comprises a DNA or RNA segment of HIV.

**36.** The method of claim 1 used to detect the presence of HCV in a biological sample in which the analyte comprises a DNA or RNA segment of HCV.

**37.** A method of amplifying the signal used to detect and quantify a ligand receptor in an assay comprising the following steps:

(a) immobilizing said ligand receptor directly or indirectly on a solid phase;
(b) binding to the ligand receptor a ligand specific for said receptor, said ligand being coupled to an oligonucleotide as defined in claim 1;
(c) removing unbound ligand;
(d) hybridizing a polydeoxynucleotide construct as defined in claim 1 to the oligonucleotide;
(e) removing unhybridized constructs;
(f) transcribing multiple copies of RNA oligomers which are complementary to the template sequence, c', of the third domain, C, of said polydeoxynucleotide construct via a DNA-dependent RNA polymerase activity; and
(g) quantifying the RNA transcripts.

**38.** The hybridization assay of claim 37 in which the ligand receptor is an antigen and the ligand is an antibody which immunologically reacts with the antigen.

**39.** A method of constructing a polydeoxynucleotide construct as defined in claim 1 which comprises combining said domains A, B and C in covalent linkage in the orientation A-B-C or B-C-A.

**40.** An isolated polydeoxynucleotide construct for use as a signal amplifier in hybridization assays comprising three domains:

(a) a first domain (A) which is single-stranded and has a nucleotide sequence complementary to a target sequence;
(b) a second domain (B) which is double-stranded and capable of functioning as a promoter for a DNA-dependent RNA polymerase enzyme activity; and
(c) a third domain (C) which is either single- or double-stranded and adjacent to said second domain, such that said third domain is capable of functioning as a template for the promoter activity of said second domain, wherein the domains of the construct are oriented A-B-C or B-C-A, and further wherein the third domain does not contain target sequence.

**Patentansprüche**

**Patentansprüche für folgende Vertragsstaaten : AT, BE, CH, LI, DE, DK, FR, GB, GR, IT, LU, NL, SE**

**1.** Isoliertes Polydesoxynukleotidkonstrukt zur Verwendung als Signalamplifizierungsmittel in Hybridisierungsassays, umfassend drei Domänen:

(a) eine erste Domäne (A), die einzelsträngig ist und die eine Nukleotidsequenz besitzt, die komplementär zu einer Zielsequenz ist;
(b) eine zweite Domäne (B), die doppelsträngig ist und in der Lage ist, als Promotor für eine DNA-abhängige RNA-Polymeraseenzymaktivität zu wirken; und
(c) eine dritte Domäne (C), die entweder einzel- oder doppelsträngig ist und neben der zweiten Domäne liegt, so daß die dritte Domäne in der Lage ist, als Matrize für die Promotoraktivität der zweiten Domäne zu wirken, wobei die Domänen des Konstrukts in der Orientierung A-B-C oder B-C-A vorliegen und wobei weiterhin die dritte Domäne keine Zielsequenz enthält.

**2.** Polydesoxynukleotidkonstrukt nach Anspruch 1, bei dem der genannte Promotor in der Lage ist, als Promotor für eine DNA-abhängige RNA-Polymerase, die vom Bakteriophagen T7 abgeleitet ist, zu wirken.

**3.** Polydesoxynukleotidkonstrukt nach Anspruch 1, bei dem der genannte Promotor in der Lage ist, als Promotor für die DNA-abhängige RNA-Polymerase, die vom Bakteriophagen T3 abgeleitet ist, zu wirken.

4. Polydesoxynukleotidkonstrukt nach Anspruch 1, bei dem der Promotor in der Lage ist, als Promotor für die DNA-abhängige RNA-Polymerase, abgeleitet vom Bakteriophagen SP6, zu wirken.

5. Polydesoxynukleotidkonstrukt nach Anspruch 1, bei dem die erste Domäne A wenigstens 10 und nicht mehr als 40 Nukleotide lang ist.

6. Polydesoxynukleotidkonstrukt nach Anspruch 1, bei dem die genannte erste Domäne A wenigstens 15 und nicht mehr als 30 Nukleotide lang ist.

7. Polydesoxynukleotidkonstrukt nach Anspruch 5, bei dem die genannte zweite Domäne B wenigstens 12 und nicht mehr als 40 Nukleotide lang ist.

8. Polydesoxynukleotidkonstrukt nach Anspruch 5, bei dem die genannte zweite Domäne B wenigstens 17 und nicht mehr als 30 Nukleotide lang ist.

9. Polydesoxynukleotidkonstrukt nach Anspruch 7, bei dem die genannte dritte Domäne C wenigstens 30 und nicht mehr als 10.000 Nukleotide lang ist.

10. Polydesoxynukleotidkonstrukt nach Anspruch 7, bei dem die genannte dritte Domäne C wenigstens 40 und nicht mehr als 80 Nukleotide lang ist.

11. Polydesoxynukleotidkonstrukt nach Anspruch 7, bei dem die genannte dritte Domäne C eine Länge von wenigstens 2 kB und nicht mehr als 10 kB besitzt.

12. Polydesoxynukleotidkonstrukt nach Anspruch 7, bei dem die genannte dritte Domäne C eine Länge von wenigstens 3 kB und nicht mehr als 4 kB besitzt.

13. Polydesoxynukleotidkonstrukt nach Anspruch 7, bei dem die genannte dritte Domäne C im wesentlichen das ganze Genom des Hepatitis-B-Virus umfaßt.

14. Polydesoxynukleotidkonstrukt nach Anspruch 2, bei dem die Sequenz der genannten zweiten Domäne B die folgende Sequenz umfaßt:

```
5'-TAA TAC GAC TCA CTA TA-3'
3'-ATT ATG CTG AGT GAT AT-5'
```

15. Polydesoxynukleotidkonstrukt nach Anspruch 2, bei dem die Nukleotidsequenz der genannten zweiten Domäne B wie folgt ist:

```
5'-CTG GCT TAT CGA AAT TAA TAC GAC TCA CTA TA-3'
3'-GAC CGA ATA GCT TTA ATT ATG CTG AGT GAT AT-5'
```

16. Polydesoxynukleotidkonstrukt nach Anspruch 1, bei dem der 5'-Rest des oberen Stranges der Nukleotidsequenz der genannten dritten Domäne C im Anschluß an die genannte zweite Domäne B liegt und ein Guanosinrest ist.

17. Polydesoxynukleotidkonstrukt nach Anspruch 1, bei dem die Sequenz für die genannte dritte Domäne C wie folgt ist:

```
5'-GGG AGA TGT GGT TGT CGT ACT TAG CGA AAT ACT GTC CGA
GTC G-3'
3'-CCC TCT ACA CCA ACA GCA TGA ATC GCT TTA TGA CAG GCT
CAG C-5'
```

18. Polydesoxynukleotidkonstrukt nach Anspruch 17, bei dem das 3'-Ende des oberen Stranges der DNA-Nukleotidsequenz der genannten zweiten Domäne B an das 5'-Ende des oberen Stranges der genannten Nukleotidsequenz der Domäne C gebunden ist.

19. Polydesoxynukleotidkonstrukt nach Anspruch 1, bei dem das Transkript der genannten dritten Domäne C zwei Subdomänen besitzt:

(a) eine erste Subdomäne $c_1$, die in der Lage ist, an einen Oligonukleotideinfanglinker zu hybridisieren, wobei der Einfanglinker in der Lage ist, an ein Polynukleotid zu hybridisieren, das an ein festes Substrat immobilisiert ist; und
(b) eine zweite Subdomäne $c_2$, die in der Lage ist, an einen Oligonukleotidamplifizierungslinker zu binden, wobei der Amplifizierungslinker in der Lage ist, an eine quantifizierbare Sonde zu binden.

20. Polydesoxynukleotidkonstrukt nach Anspruch 1, bei dem die ersten und dritten Domänen, B und C, in mehrfach wiederholenden Einheiten vorhanden sind.

21. Verfahren zur Amplifikation eines Signals, das verwendet wird, um einen Oligonukleotidanalyten in einem Hybridisierungsassay nachzuweisen und zu quantifizieren, umfassend die folgenden Stufen:

(i) direkte oder indirekte Immobilisierung des Analyten an ein festes Substrat und direkte oder indirekte Hybridisierung des Polydesoxynukleotidkonstrukts nach Anspruch 1 an den Analyten,
(ii) Entfernung nichthybridisierter Polydesoxynukleotidkonstrukte;
(iii) Transkribieren multipler Kopien von RNA-Oligomeren, die komplementär zu der Matrizensequenz, c', der genannten dritten Domäne C des genannten Polydesoxynukleotidkonstrukts sind, über eine DNA-abhängige RNA-Polymeraseaktivität; und
(iv) Nachweis der Menge von RNA-Transkripten, die in der Stufe (iii) gebildet wurden.

22. Nukleinsäure-Hybridisierungsassay nach Anspruch 21, bei dem die genannte zweite Domäne B einen Promotor für die DNA-abhängige RNA-Polymerase des Bakteriophagen T7 bereitstellt.

23. Nukleinsäure-Hybridisierungsassay nach Anspruch 21, bei dem die genannte zweite Domäne B einen Promotor für die DNA-abhängige RNA-Polymerase des Bakteriophagen T3 bereitstellt.

24. Nukleinsäure-Hybridisierungsassay nach Anspruch 21, bei dem die genannte zweite Domäne B einen Promotor für die DNA-abhängige RNA-Polymerase des Bakteriophagen SP6 bereitstellt.

25. Nukleinsäure-Hybridisierungsassay nach Anspruch 21, bei dem das Polydesoxynukleotidkonstrukt direkt an einen einzelsträngigen Analyten hybridisiert wird.

26. Nukleinsäure-Hybridisierungsassay nach Anspruch 21, bei dem das Polydesoxynukleotidkonstrukt an einen Oligonukleotidlinker hybridisiert wird, und wobei der Linker eine Domäne enthält, die in der Lage ist, stabile Hybride mit dem Analyten zu bilden.

27. Nukleinsäure-Hybridisierungsassay nach Anspruch 21, bei dem:

(a) die genannte dritte Domäne C des Polydesoxynukleotidkonstrukts in Gegenwart markierter Ribonukleotidtriphosphate transkribiert wird;
(b) die Transkripte der genannten dritten Domäne eine erste Subdomäne, $c_1$, besitzen, die komplementär zu einer Oligonukleotideinfangsonde, die auf einem festen Substrat immobilisiert ist, ist; und

(c) die Transkripte immobilisiert und quantifiziert werden.

**28.** Nukleinsäure-Hybridisierungsassay nach Anspruch 21, bei dem:

(a) die genannte dritte Domäne C des Polydesoxynukleotidkonstrukts in Gegenwart biotinylierter Ribonukleotidtriphosphate transkribiert wird;
(b) das Transkript der genannten dritten Domäne eine erste Subdomäne, $c_2$, die komplementär zu einer markierten Oligonukleotidsonde ist, besitzt; und
(c) die Transkripte auf einem avidinylierten festen Träger immobilisiert werden; und
(d) die Transkripte quantifiziert werden.

**29.** Nukleinsäure-Hybridisierungsassay nach Anspruch 21, bei dem:

(a) die genannte dritte Domäne C des Polydesoxynukleotidkonstrukts in Gegenwart sowohl markierter als auch biotinylierter Ribonukleotidtriphosphate transkribiert wird;
(b) die Transkripte auf einem avidinylierten festen Substrat immobilisiert werden; und
(c) die Transkripte quantifiziert werden.

**30.** Nukleinsäure-Hybridisierungsassay nach Anspruch 21, bei dem:

(a) das Transkript der genannten dritten Domäne C des Polydesoxynukleotidkonstrukts zwei Subdomänen besitzt:

(i) eine erste Subdomäne, $c_1$, die komplementär zu einer Oligonukleotideinfangsonde ist, wobei die Sonde auf einem festen Substrat immobilisiert ist; und
(ii) eine zweite Subdomäne, $c_2$, die komplementär zu einer markierten Oligonukleotidsonde ist;

(b) das Transkript an die Einfangsonde hybridisiert wird;
(c) die markierte Sonde an die Transkripte hybridisiert wird; und
(d) die Transkripte quantifiziert werden.

**31.** Nukleinsäure-Hybridisierungsassay nach Anspruch 21, bei dem:

(a) das Transkript der genannten dritten Domäne C des Polydesoxynukleotidkonstrukts zwei Subdomänen besitzt:

(i) eine erste Subdomäne, $c_1$, die komplementär zu einer Transkripteinfangsonde ist, wobei die Transkripteinfangsonde in der Lage ist, an ein Oligonukleotid, das auf einem festen Substrat immobilisiert wurde, zu hybridisieren; und
(ii) eine zweite Subdomäne, $c_2$, die komplementär zu einer Linkersonde ist, wobei die Linkersonde in der Lage ist, an ein Markierungsoligonukleotid zu hybridisieren;

(b) das Transkript an die Transkripteinfangsonde und die Linkersonde hybridisiert wird, um ein Transkriptsandwich zu bilden;
(c) das Transkriptsandwich an ein Oligonukleotid hybridisiert wird, das auf einem festen Substrat immobilisiert ist;
(d) das immobilisierte Sandwich an ein Markierungsoligonukleotid hybridisiert wird; und
(e) die Transkripte quantifiziert werden.

**32.** Nukleinsäure-Hybridisierungsassay nach Anspruch 21, bei dem:

(a) das Transkript der genannten dritten Domäne C des Polydesoxynukleotidkonstrukts zwei Subdomänen besitzt;

(i) eine erste Subdomäne, $c_1$, die komplementär zu einer Transkripteinfangsonde ist, wobei die Transkripteinfangsonde in der Lage ist, an ein Oligonukleotid zu hybridisieren, das auf einem festen Substrat immobilisiert wurde; und
(ii) eine zweite Subdomäne, $c_2$, die komplementär zu einer Amplifizierungslinkersonde ist, wobei die Linkersonde in der Lage ist, mit einer Amplifizierungssonde zu hybridisieren;

(b) das Transkript an die Transkripteinfangsonde und die Amplifizierungslinkersonde hybridisiert wird, um ein Transkriptsandwich zu bilden;

(c) das Transkriptsandwich an ein Oligonukleotid hybridisiert wird, das auf einem festen Substrat immobilisiert wurde;

(d) das immobilisierte Sandwich mit einer Amplifizierungssonde hybridisiert wird;

(e) die Amplifizierungssonde mit einem Markierungsoligonukleotid hybridisiert wird; und

(f) die Transkripte quantifiziert werden.

33. Nukleinsäure-Hybridisierungsassay nach Anspruch 32, bei dem das RNA-Transkript von der DNA des Hepatitis-B-Virus transkribiert wird.

34. Nukleinsäure-Hybridisierungsassay nach Anspruch 21, bei dem die genannten zweiten und dritten Domänen B und C des Polydesoxynukleotidkonstrukts in multiplen wiederholenden Einheiten vorhanden sind.

35. Verfahren nach Anspruch 21, verwendet zum Nachweis des Vorhandenseins von N. gonorrhoeae in einer biologischen Probe, bei dem der Analyt ein DNA- oder RNA-Segment von N. gonorrhoeae enthält.

36. Verfahren nach Anspruch 21, verwendet zum Nachweis des Vorhandenseins von Hepatitis-B-Virus in einer biologischen Probe, bei dem der Analyt ein DNA- oder RNA-Segment des Hepatitis-B-Virus enthält.

37. Verfahren nach Anspruch 21, verwendet zum Nachweis des Vorhandenseins von Bakterien, enthaltend das β-Lactamase TEM-1-Gen in einer biologischen Probe, bei dem der Analyt ein DNA- oder RNA-Segment des β-Lactamase TEM-1-Gens enthält.

38. Verfahren nach Anspruch 21, verwendet zum Nachweis des Vorhandenseins von Chlamydia in einer biologischen Probe, bei dem der Analyt ein DNA- oder RNA-Segment von Chlamydia enthält.

39. Verfahren nach Anspruch 21, verwendet zum Nachweis des Vorhandenseins von Bakterien, enthaltend die tet-M-Determinante in einer biologischen Probe, bei dem der Analyt ein DNA- oder RNA-Segment der tet-M-Determinante enthält.

40. Verfahren nach Anspruch 21, verwendet zum Nachweis des Vorhandenseins von HIV in einer biologischen Probe, bei dem der Analyt ein DNA- oder RNA-Segment von HIV umfaßt.

41. Verfahren nach Anspruch 21, verwendet zum Nachweis des Vorhandenseins von HCV in einer biologischen Probe, bei dem der Analyt ein DNA- oder RNA-Segment von HCV enthält.

42. Verfahren zur Amplifikation des Signals, das zum Nachweis und zur Quantifizierung eines Ligandenrezeptors in einem Assay verwendet wird, umfassend die folgenden Stufen:

(a) Immobilisierung des Ligandenrezeptors direkt oder indirekt auf einer Festphase;

(b) Bindung eines Liganden, der für diesen Rezeptor spezifisch ist, an den Ligandenrezeptor, wobei der Ligand an ein Oligonukleotid gekoppelt ist, das komplementär zur ersten Domäne des Konstrukts nach Anspruch 1 ist;

(c) Entfernung von nichtgebundenem Liganden;

(d) Hybridisierung des Konstrukts nach Anspruch 1 an das Oligonukleotid;

(e) Entfernung nichthybridisierter Konstrukte;

(f) Transkription multipler Kopien von RNA-Oligomeren, die komplementär zu der Matrizensequenz, c', der dritten Domäne C des Polydesoxynukleotidkonstrukts sind, über eine DNA-abhängige RNA-Polymeraseaktivität; und

(g) Quantifizierung der RNA-Transkripte.

43. Hybridisierungsassay nach Anspruch 42, bei dem der Ligandenrezeptor ein Antigen und der Ligand ein Antikörper ist, der immunologisch mit dem Antigen reagiert.

# EP 0 510 085 B1

**Patentansprüche für folgenden Vertragsstaat : ES**

1. Verfahren zur Amplifikation eines Signals, verwendet zum Nachweis und zur Quantifikation eines Oligonukleotidanalyten in einem Hybridisierungsassay, umfassend die folgenden Stufen:

   (i) direkte oder indirekte Immobilisierung des Analyten auf einem festen Substrat und direkte oder indirekte Hybridisierung eines Polydesoxynukleotidkonstrukts mit dem Analyten, wobei das Polydesoxynukleotidkonstrukt die folgenden drei Domänen enthält:

   (a) eine erste Domäne (A), die einzelsträngig ist und die eine Nukleotidsequenz besitzt, die komplementär zu einer Zielsequenz ist;
   (b) eine zweite Domäne (B), die doppelsträngig ist und in der Lage ist, als Promotor für eine DNA-abhängige RNA-Polymeraseenzymaktivität zu wirken; und
   (c) eine dritte Domäne (C), die entweder einzelsträngig oder doppelsträngig ist und im Anschluß an die genannte zweite Domäne liegt, so daß die genannte dritte Domäne in der Lage ist, als Matrize für die Promotoraktivität der genannten zweiten Domäne zu wirken,

   worin die Domänen des Konstrukts in der A-B-C- oder B-C-A-Orientierung vorliegen und wobei weiter die dritte Domäne keine Zielsequenz enthält;
   (ii) Entfernung nichthybridisierter Polydesoxynukleotidkonstrukte;
   (iii) Transkribieren multipler Kopien von RNA-Oligomeren, die komplementär zu der Matrizensequenz c' der genannten dritten Domäne C des Polydesoxynukleotidkonstrukts sind, über eine DNA-abhängige RNA-Polymeraseaktivität; und
   (iv) Nachweis der Menge von RNA-Transkripten, die in der Stufe (iii) gebildet wurden.

2. Nukleinsäure-Hybridisierungsassay nach Anspruch 1, bei dem die genannte zweite Domäne B einen Promotor für die DNA-abhängige RNA-Polymerase des Bakteriophagen T7 bereitstellt.

3. Nukleinsäure-Hybridisierungsassay nach Anspruch 1, bei dem die genannte zweite Domäne B einen Promotor für die DNA-abhängige RNA-Polymerase des Bakteriophagen T3 bereitstellt.

4. Nukleinsäure-Hybridisierungsassay nach Anspruch 1, bei dem die genannte zweite Domäne B einen Promotor für die DNA-abhängige RNA-Polymerase des Bakteriophagen SP6 bereitstellt.

5. Nukleinsäure-Hybridisierungsassay nach einem der Ansprüche 1 bis 4, wobei die genannte erste Domäne A des Polydesoxynukleotidkonstrukts wenigstens 10 und nicht mehr als 40 Nukleotide lang ist.

6. Nukleinsäure-Hybridisierungsassay nach Anspruch 5, bei dem die genannte erste Domäne A wenigstens 15 und nicht mehr als 30 Nukleotide lang ist.

7. Nukleinsäure-Hybridisierungsassay nach Anspruch 5, bei dem die genannte zweite Domäne B des genannten Polydesoxynukleotidkonstrukts wenigstens 12 und nicht mehr als 40 Nukleotide lang ist.

8. Nukleinsäure-Hybridisierungsassay nach Anspruch 7, bei dem die genannte zweite Domäne B wenigstens 17 und nicht mehr als 30 Nukleotide lang ist.

9. Nukleinsäure-Hybridisierungsassay nach Anspruch 7, bei dem die dritte Domäne C des Polydesoxynukleotidkonstrukts wenigstens 30 und nicht mehr als 10.000 Nukleotide lang ist.

10. Nukleinsäure-Hybridisierungsassay nach Anspruch 7, bei dem die genannte dritte Domäne C des Polydesoxynukleotidkonstrukts wenigstens 40 und nicht mehr als 80 Nukleotide lang ist.

11. Nukleinsäure-Hybridisierungsassay nach Anspruch 7, bei dem die genannte dritte Domäne C des Polynukleotidkonstrukts eine Länge von wenigstens 2 kB und nicht mehr als 10 kB besitzt.

12. Nukleinsäure-Hybridisierungsassay nach Anspruch 7, bei dem die genannte dritte Domäne C des Polynukleotidkonstrukts eine Länge von wenigstens 3 kB und nicht mehr als 4 kB besitzt.

13. Nukleinsäure-Hybridisierungsassay nach Anspruch 7, bei dem die genannte dritte Domäne C des Polynukleotidkonstrukts im wesentlichen das ganze Genom des Hepatitis-B-Virus enthält.

14. Nukleinsäure-Hybridisierungsassay nach Anspruch 2, bei dem die Sequenz der genannten zweiten Domäne B die folgende Sequenz umfaßt:

$$5'-TAA\ TAC\ GAC\ TCA\ CTA\ TA-3'$$
$$3'-ATT\ ATG\ CTG\ AGT\ GAT\ AT-5'$$

15. Nukleinsäure-Hybridisierungsassay nach Anspruch 2, bei dem die Nukleotidsequenz der genannten zweiten Domäne B wie folgt ist:

$$5'-CTG\ GCT\ TAT\ CGA\ AAT\ TAA\ TAC\ GAC\ TCA\ CTA\ TA-3'$$

$$3'-GAC\ CGA\ ATA\ GCT\ TTA\ ATT\ ATG\ CTG\ AGT\ GAT\ AT-5'$$

16. Nukleinsäure-Hybridisierungsassay nach Anspruch 1, bei dem der 5'-Rest des oberen Stranges der Nukleotidsequenz der genannten dritten Domäne C im Anschluß an die genannte zweite Domäne B liegt und ein Guanosinrest ist.

17. Nukleinsäure-Hybridisierungsassay nach Anspruch 1, bei dem die Sequenz für die genannte dritte Domäne C wie folgt ist:

$$5'-GGG\ AGA\ TGT\ GGT\ TGT\ CGT\ ACT\ TAG\ CGA\ AAT\ ACT\ GTC\ CGA$$
$$GTC\ G-3'$$
$$3'-CCC\ TCT\ ACA\ CCA\ ACA\ GCA\ TGA\ ATC\ GCT\ TTA\ TGA\ CAG\ GCT$$
$$CAG\ C-5'$$

18. Nukleinsäure-Hybridisierungsassay nach Anspruch 17, bei dem das 3'-Ende des oberen Stranges der DNA-Nukleotidsequenz der genannten zweiten Domäne B an das 5'-Ende des oberen Stranges der genannten Nukleotidsequenz der Domäne C gebunden ist.

19. Nukleinsäure-Hybridisierungsassay nach Anspruch 1, bei dem das Transkript der genannten dritten Domäne C zwei Subdomänen besitzt:

(a) eine erste Subdomäne $c_1$, die in der Lage ist, an einen Oligonukleotideinfanglinker zu hybridisieren, wobei der Einfanglinker in der Lage ist, an ein Polynukleotid zu hybridisieren, das an ein festes Substrat immobilisiert ist; und
(b) eine zweite Subdomäne $c_2$, die in der Lage ist, an einen Oligonukleotidamplifizierungslinker zu binden, wobei der Amplifizierungslinker in der Lage ist, an eine quantifizierbare Sonde zu binden.

20. Nukleinsäure-Hybridisierungsassay nach Anspruch 1, bei dem die zweiten und dritten Domänen, B und C des Polydescxynuklectidkonstrukts, in mehrfach wiederholenden Einheiten vorhanden sind.

21. Nukleinsäure-Hybridisierungsassay nach Anspruch 1, bei dem das Polydesoxynukleotidkonstrukt direkt an einen einzelsträngigen Analyten hybridisiert wird.

**22.** Nukleinsäure-Hybridisierungsassay nach Anspruch 1, bei dem das Polydesoxynukleotidkonstrukt an einen Oligonukleotidlinker hybridisiert wird, wobei der Linker eine Domäne besitzt, die in der Lage ist, stabile Hybride mit dem Analyten zu bilden.

**23.** Nukleinsäure-Hybridisierungsassay nach Anspruch 1, bei dem:

(a) die genannte dritte Domäne C des Polydesoxynukleotidkonstrukts in Gegenwart von markierten Ribonukleotidtriphosphaten transkribiert wird;
(b) die Transkripte der genannten dritten Domäne eine erste Subdomäne $c_1$, komplementär zu einer Oligonukleotideinfangsonde, die auf einem festen Substrat immobilisiert wurde, besitzen; und
(c) die Transkripte immobilisiert und quantifiziert werden.

**24.** Nukleinsäure-Hybridisierungsassay nach Anspruch 1, bei dem:

(a) die genannte dritte Domäne C des Polydesoxynukleotidkonstrukts in Gegenwart biotinylierter Ribonukleotidtriphosphate transkribiert wird;
(b) das Transkript der genannten dritten Domäne eine erste Subdomäne, $c_2$, die komplementär zu einer markierten Oligonukleotidsonde ist, besitzt; und
(c) die Transkripte auf einem avidinylierten festen Substrat immobilisiert werden; und
(d) die Transkripte quantifiziert werden.

**25.** Nukleinsäure-Hybridisierungsassay nach Anspruch 1, bei dem:

(a) die genannte dritte Domäne C des Polydesoxynukleotidkonstrukts in Gegenwart sowohl markierter als auch biotinylierter Ribonukleotidtriphosphate transkribiert wird;
(b) die Transkripte auf einem avidinylierten festen Substrat immobilisiert werden; und
(c) die Transkripte quantifiziert werden.

**26.** Nukleinsäure-Hybridisierungsassay nach Anspruch 1, bei dem:

(a) das Transkript der genannten dritten Domäne C des Polydesoxynukleotidkonstrukts zwei Subdomänen besitzt:

(i) eine erste Subdomäne, $c_1$, die komplementär zu einer Oligonukleotideinfangsonde ist, wobei die Sonde auf einem festen Substrat immobilisiert ist; und
(ii) eine zweite Subdomäne, $c_2$, die komplementär zu einer markierten Oligonukleotidsonde ist;

(b) das Transkript an die Einfangsonde hybridisiert wird;
(c) die markierte Sonde an die Transkripte hybridisiert wird; und
(d) die Transkripte quantifiziert werden.

**27.** Nukleinsäure-Hybridisierungsassay nach Anspruch 1, bei dem:

(a) das Transkript der genannten dritten Domäne C des Polydesoxynukleotidkonstrukts zwei Subdomänen besitzt:

(i) eine erste Subdomäne, $c_1$, die komplementär zu einer Transkripteinfangsonde ist, wobei die Transkripteinfangsonde in der Lage ist, an ein Oligonukleotid, das auf einem festen Substrat immobilisiert wurde, zu hybridisieren; und
(ii) eine zweite Subdomäne, $c_2$, die komplementär zu einer Linkersonde ist, wobei die Linkersonde in der Lage ist, an ein Markierungsoligonukleotid zu hybridisieren;

(b) das Transkript an die Transkripteinfangsonde und die Linkersonde hybridisiert wird, um ein Transkriptsandwich zu bilden;
(c) das Transkriptsandwich an ein Oligonukleotid hybridisiert wird, das auf einem festen Träger immobilisiert ist;
(d) das immobilisierte Sandwich an ein Markierungsoligonukleotid hybridisiert wird; und

(e) die Transkripte quantifiziert werden.

**28.** Nukleinsäure-Hybridisierungsassay nach Anspruch 1, bei dem:

(a) das Transkript der genannten dritten Domäne C des Polydesoxynukleotidkonstrukts zwei Subdomänen besitzt;

(i) eine erste Subdomäne, $c_1$, die komplementär zu einer Transkripteinfangsonde ist, wobei die Transkripteinfangsonde in der Lage ist, an ein Oligonukleotid zu hybridisieren, das auf einem festen Substrat immobilisiert wurde; und
(ii) eine zweite Subdomäne, $c_2$, die komplementär zu einer Amplifizierungslinkersonde ist, wobei die Linkersonde in der Lage ist, mit einer Amplifizierungssonde zu hybridisieren;

(b) das Transkript an die Transkripteinfangscnde und die Amplifizierungslinkersonde hybridisiert wird, um ein Transkriptsandwich zu bilden;
(c) das Transkriptsandwich an ein Oligonukleotid hybridisiert wird, das auf einem festen Substrat immobilisiert wurde;
(d) das immobilisierte Sandwich mit einer Amplifizierungssonde hybridisiert wird;
(e) die Amplifizierungssonde mit einem Markierungsoligonukleotid hybridisiert wird; und
(f) die Transkripte quantifiziert werden.

**29.** Nukleinsäure-Hybridisierungsassay nach Anspruch 28, bei dem das RNA-Transkript von der DNA des Hepatitis-B-Virus transkribiert wird.

**30.** Verfahren nach Anspruch 1, verwendet zum Nachweis des Vorhandenseins von N. gonorrhoeae in einer biologischen Probe, bei dem der Analyt ein DNA- cder RNA-Segment von N. gonorrhoeae umfaßt.

**31.** Verfahren nach Anspruch 1, verwendet zum Nachweis des Vorhandenseins von Hepatitis-B-Virus in einer biologischen Probe, bei dem der Analyt ein DNA- oder RNA-Segment des Hepatitis-B-Virus umfaßt.

**32.** Verfahren nach Anspruch 1, verwendet zum Nachweis des Vorhandenseins von Bakterien, enthaltend das $\beta$-Lactamase TEM-1-Gen in einer biologischen Probe, bei dem der Analyt ein DNA- oder RNA-Segment des $\beta$-Lactamase TEM-1-Gens enthält.

**33.** Verfahren nach Anspruch 1, verwendet zum Nachweis des Vorhandenseins von Chlamydia in einer biologischen Probe, bei dem der Analyt ein DNA- oder RNA-Segment von Chlamydia enthält.

**34.** Verfahren nach Anspruch 1, verwendet zum Nachweis des Vorhandenseins von Bakterien, enthaltend die tet-M-Determinante in einer biologischen Probe, bei dem der Analyt ein DNA- oder RNA-Segment der tet-M-Determinante enthält.

**35.** Verfahren nach Anspruch 1, verwendet zum Nachweis des Vorhandenseins von HIV in einer biologischen Probe, bei dem der Analyt ein DNA- oder RNA-Segment vcn HIV enthält.

**36.** Verfahren nach Anspruch 1, verwendet zum Nachweis des Vorhandenseins von HCV in einer biologischen Probe, bei dem der Analyt ein DNA- oder RNA-Segment von HCV enthält.

**37.** Verfahren zur Amplifikation des Signals, das zum Nachweis und zur Quantifikation eines Ligandenrezeptors in einem Assay verwendet wird, umfassend die folgenden Stufen:

(a) Immobilisierung des Ligandenrezeptors direkt oder indirekt auf einer Festphase;
(b) Bindung eines Liganden, der für diesen Rezeptor spezifisch ist, an den Ligandenrezeptor, wobei der Ligand an ein Oligonukleotid wie in Anspruch 1 definiert gekoppelt ist;
(c) Entfernung von nichtgebundenem Liganden;
(d) Hybridisierung des Polydesoxynukleotidkonstrukts nach Anspruch 1 an das Oligonukleotid;
(e) Entfernung nichthybridisierter Konstrukte;
(f) Transkription multipler Kopien von RNA-Oligomeren, die komplementär zu der Matrizensequenz c' der dritten Domäne C des Polydesoxynukleotidkonstrukts sind, über eine DNA-abhängige RNA-Polymeraseaktivität; und

(g) Quantifizierung der RNA-Transkripte.

**38.** Hybridisierungsassay nach Anspruch 37, bei dem der Ligandenrezeptor ein Antigen und der Ligand ein Antikörper, der mit dem Antigen immunologisch reagiert, sind.

**39.** Verfahren zur Konstruktion eines Polydesoxynukleotidkonstrukts, wie in Anspruch 1 definiert, umfassend die Kombination der genannten Domänen A, B und C in covalenter Verknüpfung in der Orientierung A-B-C oder B-C-A.

**40.** Isoliertes Polydesoxynukleotidkonstrukt zur Verwendung als Signalamplifikationsmittel in einem Hybridisierangsassay, umfassend drei Domänen:

(a) eine erste Domäne (A), die einzelsträngig ist und eine Nukleotidsequenz besitzt, die komplementär zu einer Zielsequenz ist;

(b) eine zweite Domäne (B), die doppelsträngig ist und in der Lage ist als Promotor für eine DNA-abhängige RNA-Polymeraseenzymaktivität zu wirken; und

(c) eine dritte Domäne (C), die entweder einzelsträngig oder doppelsträngig ist und im Anschluß an die genannte zweite Domäne liegt, so daß die genannte dritte Domäne in der Lage ist, als Matrize für die Promotoraktivität der genannten zweiten Domäne zu wirken, wobei die Domänen des Konstrukts in der A-B-C- oder B-C-A-Orientierung vorliegen und wobei die dritte Domäne weiterhin keine Zielsequenz enthält.

## Revendications

**Revendications pour les Etats contractants suivants : AT, BE, CH, LI, DE, DK, FR, GB, GR, IT, LU, NL, SE**

**1.** Construction polydésoxynucléotidique isolée destinée à une utilisation comme amplificateur de signal dans des tests d'hybridation comprenant trois domaines :

(a) un premier domaine (A) qui est simple brin et a une séquence nucléotidique complémentaire d'une séquence cible ;

(b) un deuxième domaine (B) qui est double brin et est capable de fonctionner comme promoteur de l'activité enzymatique d'une ARN-polymérase ADN-dépendante ; et

(c) un troisième domaine (C) qui est soit simple brin soit double brin et adjacent audit deuxième domaine, de sorte que ledit troisième domaine est capable de fonctionner comme matrice pour l'activité promotrice dudit deuxième domaine, dans laquelle les domaines de la construction sont orientés A-B-C ou B-C-A et dans laquelle, en outre, le troisième domaine ne contient pas de séquence cible.

**2.** Construction polydésoxynucléotidique selon la revendication 1 dans laquelle ledit promoteur est capable de fonctionner comme promoteur pour l'ARN-polymérase ADN-dépendante provenant du bactériophage T7.

**3.** Construction polydésoxynucléotidique selon la revendication 1 dans laquelle ledit promoteur est capable de fonctionner comme promoteur pour l'ARN-polymérase ADN-dépendante provenant du bactériophage T3.

**4.** Construction polydésoxynucléotidique selon la revendication 1 dans laquelle ledit promoteur est capable de fonctionner comme promoteur pour l'ARN-polymérase ADN-dépendante provenant du bactériophage SP6.

**5.** Construction polydésoxynucléotidique selon la revendication 1 dans laquelle ledit premier domaine A a une longueur d'au moins 10 et pas plus de 40 nucléotides.

**6.** Construction polydésoxynucléotidique selon la revendication 1 dans laquelle ledit premier domaine A a une longueur d'au moins 15 et pas plus de 30 nucléotides.

**7.** Construction polydésoxynucléotidique selon la revendication 5 dans laquelle ledit deuxième domaine B a une longueur d'au moins 12 et pas plus de 40 nucléotides.

8. Construction polydésoxynucléotidique selon la revendication 5 dans laquelle ledit deuxième domaine B a une longueur d'au moins 17 et pas plus de 30 nucléotides.

9. Construction polydésoxynucléotidique selon la revendication 7 dans laquelle ledit troisième domaine C a une longueur d'au moins 30 et pas plus de 10 000 nucléotides.

10. Construction polydésoxynucléotidique selon la revendication 7 dans laquelle ledit troisième domaine C a une longueur d'au moins 40 et pas plus de 80 nucléotides.

11. Construction polydésoxynucléotidique selon la revendication 7 dans laquelle ledit troisième domaine C a une longueur d'au moins 2 Kb et pas plus de 10 Kb.

12. Construction polydésoxynucléotidique selon la revendication 7 dans laquelle ledit troisième domaine C a une longueur d'au moins 3 Kb et pas plus de 4 Kb.

13. Construction polydésoxynucléotidique selon la revendication 7 dans laquelle ledit troisième domaine C est constitué d'essentiellement tout le génome du virus de l'hépatite B.

14. Construction polydésoxynucléotidique selon la revendication 2 dans laquelle la séquence dudit deuxième domaine B comprend la séquence :

```
5'-TAA TAC GAC TCA CTA TA-3'
3'-ATT ATG CTG AGT GAT AT-5'  .
```

15. Construction polydésoxynucléotidique selon la revendication 2 dans laquelle la séquence nucléotidique dudit deuxième domaine B est :

```
5'-CTG GCT TAT CGA AAT TAA TAC GAC TCA CTA TA-3'
3'-GAC CGA ATA GCT TTA ATT ATG CTG AGT GAT AT-5'.
```

16. Construction polydésoxynucléotidique selon la revendication 1 dans laquelle le résidu en 5' du brin supérieur de la séquence nucléotidique dudit troisième domaine C est adjacent audit deuxième domaine B et est un résidu guanosine.

17. Construction polydésoxynucléotidique selon la revendication 1 dans laquelle la séquence dudit troisième domaine C est :

```
5'-GGG AGA TGT GGT TGT CGT ACT TAG CGA AAT ACT GTC CGA
GTC G-3'
3'-CCC TCT ACA CCA ACA GCA TGA ATC GCT TTA TGA CAG GCT
CAG C-5'
```

18. Constructions polydésoxynucléotidique selon la revendication 17 dans lesquelles l'extrémité 3' du brin supérieur de la séquence nucléotidique de l'ADN dudit deuxième domaine B est attachée à l'extrémité 5' du brin supérieur de ladite séquence nucléotidique du domaine C.

19. Construction polydésoxynucléotidique selon la revendication 1 dans laquelle le transcrit dudit troisième domaine C possède deux sous-domaines :

(a) un premier sous-domaine, $c_1$, qui est capable de s'hybrider avec un linker de capture oligonucléotidique, ledit linker de capture étant capable de s'hybrider avec un polynucléotide immobilisé sur un substrat solide ; et

(b) un second sous-domaine, $c_2$, qui est capable de se lier à un linker d'amplification oligonucléotidique, ledit linker d'amplification étant capable de se lier à une sonde quantifiable.

20. Construction polydésoxynucléotidique selon la revendication 1 dans laquelle lesdits deuxième et troisième domaines, B et C, sont présents en unités répétées multiples.

21. Méthode d'amplification d'un signal utilisée pour détecter et quantifier un analyte oligonucléotidique dans un test d'hybridation comprenant :

(i) l'immobilisation dudit analyte, directement ou indirectement, sur un substrat solide ; et l'hybridation de la construction polydésoxynucléotidique selon la revendication 1, directement ou indirectement avec l'analyte ;
(ii) l'élimination des constructions polydésoxynucléotidiques non hybridées ;
(iii) la transcription de copies multiples d'oligomères d'ARN qui sont complémentaires de la séquence matrice , c', dudit troisième domaine, C, de ladite construction polydésoxynucléotidique via l'activité d'une ARN-polymérase ADN-dépendante ; et
(iv) la détection de la quantité de transcrits d'ARN formés à l'étape (iii).

22. Test d'hybridation d'acides nucléiques selon la revendication 21 dans lequel ledit deuxième domaine, B, fournit un promoteur pour l'ARN-polymérase ADN-dépendante du bactériophage T7.

23. Test d'hybridation d'acides nucléiques selon la revendication 21 dans lequel ledit deuxième domaine, B, fournit un promoteur pour l'ARN-polymérase ADN-dépendante du bactériophage T3.

24. Test d'hybridation d'acides nucléiques selon la revendication 21 dans lequel ledit deuxième domaine B fournit un promoteur pour l'ARN-polymérase ADN-dépendante du bactériophage SP6.

25. Test d'hybridation d'acides nucléiques selon la revendication 21 dans lequel ladite construction polydésoxynucléotidique est directement hybridée avec un analyte simple brin.

26. Test d'hybridation d'acides nucléiques selon la revendication 21 dans lequel ladite construction polydésoxynucléotidique est hybridée avec un linker oligonucléotidique, ledit linker ayant un domaine qui est capable de former des hybrides stables avec l'analyte.

27. Test d'hybridation d'acides nucléiques selon la revendication 21 dans lequel :

(a) ledit troisième domaine, C, de ladite construction polydésoxynucléotidique est transcrit en présence de ribonucléotides triphosphates marqués ;
(b) les transcrits dudit troisième domaine ont un premier sous-domaine, $c_1$, complémentaire d'une sonde de capture oligonucléotidique qui est immobilisée sur un substrat solide ; et
(c) lesdits transcrits sont immobilisés et quantifiés.

28. Test d'hybridation d'acides nucléiques selon la revendication 21 dans lequel :

(a) ledit troisième domaine, C, de ladite construction polydésoxynucléotidique est transcrit en présence de ribonucléotides triphosphates biotinylés ;
(b) ledit transcrit dudit troisième domaine a un premier sous-domaine, $c_2$, qui est complémentaire d'une sonde oligonucléotidique marquée ; et
(c) lesdits transcrits sont immobilisés sur un substrat solide avidinylé ; et
(d) lesdits transcrits sont quantifiés.

29. Test d'hybridation d'acides nucléiques selon la revendication 21 dans lequel :

(a) ledit troisième domaine,C, de ladite construction polydésoxynucléotidique est transcrit en présence à la fois de ribonucléotides triphosphates marqués et biotinylés ;
(b) lesdits transcrits sont immobilisés sur un substrat solide avidinylé ; et

(c) lesdits transcrits sont quantifiés.

30. Test d'hybridation d'acides nucléiques selon la revendication 21 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture oligonucléotidique, ladite sonde étant immobilisée sur un substrat solide ; et
(ii) un second sous-domaine, $c_2$, qui est complémentaire d'une sonde oligonucléotidique marquée ;

(b) ledit transcrit est hybridé avec ladite sonde de capture ;

(c) ladite sonde marquée est hybridée avec lesdits transcrits ; et

(d) lesdits transcrits sont quantifiés.

31. Test d'hybridation d'acides nucléiques selon la revendication 21 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture du transcrit, ladite sonde de capture du transcrit étant capable de s'hybrider avec un oligonucléotide qui a été immobilisé sur un substrat solide ; et
(ii) un deuxième domaine, $c_2$, qui est complémentaire d'une sonde linker, ladite sonde linker étant capable de s'hybrider avec un oligonucléotide marqué ;

(b) ledit transcrit est hybridé avec ladite sonde de capture du transcrit et avec ladite sonde linker pour former un transcrit en sandwich ;

(c) ledit transcrit en sandwich est hybridé avec un oligonucléotide immobilisé sur un substrat solide ;

(d) ledit sandwich immobilisé est hybridé avec un oligonucléotide de marquage ; et

(e) lesdits transcrits sont quantifiés.

32. Test d'hybridation d'acides nucléiques selon la revendication 21 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture du transcrit, ladite sonde de capture du transcrit étant capable de s'hybrider avec un oligonucléotide qui a été immobilisé sur un substrat solide ; et
(ii) un second sous-domaine, $c_2$, qui est complémentaire d'une sonde linker d'amplification, ladite sonde linker étant capable de s'hybrider avec une sonde d'amplification ;

(b) ledit transcrit est hybridé avec ladite sonde de capture du transcrit et ladite sonde linker d'amplification pour former un transcrit sandwich ;

(c) ledit transcrit sandwich est hybridé avec un oligonucléotide immobilisé sur un substrat solide ;

(d) ledit sandwich immobilisé est hybridé avec une sonde d'amplification ;

(e) ladite sonde d'amplification est hybridée avec un oligonucléotide de marquage ; et

(f) lesdits transcrits sont quantifiés.

**33.** Test d'hybridation d'acides nucléiques selon la revendication 32 dans lequel le transcrit d'ARN est transcrit à partir de l'ADN du virus de l'hépatite B.

**34.** Test d'hybridation d'acides nucléiques selon la revendication 21 dans lequel lesdits deuxième et troisième domaines, B et C, de ladite construction polydésoxynucléotidique sont présents en unités répétées multiples.

**35.** Méthode selon la revendication 21 utilisée pour détecter la présence de N. gonorrhoeae dans un échantillon biologique dans lequel l'analyte comprend un segment d'ADN ou d'ARN de N. gonorrhoeae.

**36.** Méthode selon la revendication 21 utilisée pour détecter la présence du virus de l'hépatite B dans un échantillon biologique dans lequel l'analyte comprend un segment d'ADN ou d'ARN du virus de l'hépatite B.

**37.** Méthode selon la revendication 21 utilisée pour détecter la présence de bactéries contenant le gène TEM-1 de la β-lactamase, dans un échantillon biologique dans lequel l'analyte comprend un segment d'ADN ou d'ARN du gène TEM-1 de la β-lactamase.

**38.** Méthode selon la revendication 21 utilisée pour détecter la présence de Chlamydia dans un échantillon biologique dans lequel l'analyte comprend un segment d'ADN ou d'ARN de Chlamydia.

**39.** Méthode selon la revendication 21 utilisée pour détecter la présence de bactéries contenant le déterminant tet M dans un échantillon biologique dans lequel l'analyte comprend un segment d'ADN ou d'ARN du déterminant tet M.

**40.** Méthode selon la revendication 21 utilisée pour détecter la présence de HIV dans un échantillon biologique dans lequel l'analyte comprend un segment d'ADN ou d'ARN de HIV.

**41.** Méthode selon la revendication 21 utilisée pour détecter la présence de HCV dans un échantillon biologique dans lequel l'analyte comprend un segment d'ADN ou d'ARN de HCV.

**42.** Méthode d'amplification du signal utilisée pour détecter et quantifier un récepteur de ligand dans un test comprenant les étapes suivantes :

(a) immobilisation dudit récepteur de ligand directement ou indirectement sur une phase solide ;

(b) liaison au récepteur de ligand d'un ligand spécifique dudit récepteur, ledit ligand étant couplé à un oligonucléotide complémentaire dudit premier domaine de la construction selon la revendication 1 ;

(c) élimination des ligands non liés ;

(d) hybridation de la construction selon la revendication 1 avec l'oligonucléotide ;

(e) élimination des constructions non hybridées ;

(f) transcription de copies multiples d'oligomères d'ARN qui sont complémentaires de la séquence matrice, c', du troisième domaine, C, de ladite construction polydésoxynucléotidique via l'activité d'une ARN-polymérase ADN-dépendante ; et

(g) quantification des transcrits d'ARN.

**43.** Test d'hybridation selon la revendication 42 dans lequel le récepteur de ligand est un antigène et le ligand est un anticorps qui réagit immunologiquement avec l'antigène.

**Revendications pour l'Etat contractant suivant : ES**

**1.** Méthode d'amplification d'un signal utilisée pour détecter et quantifier un analyte oligonucléotidique dans un test d'hybridation comprenant :

(i) l'immobilisation dudit analyte directement ou indirectement sur un substrat solide et l'hybridation directe ou indirecte avec ledit analyte d'une construction polydésoxynucléotidique comprenant trois domaines :

(a) un premier domaine (A) qui est simple brin et a une séquence nucléotidique complémentaire d'une séquence cible ;
(b) un deuxième domaine (B) qui est double brin et est capable de fonctionner comme promoteur de l'activité enzymatique d'une ARN-polymérase ADN-dépendante ; et
(c) un troisième domaine (C) qui est soit simple brin soit double brin et adjacent audit deuxième domaine, de sorte que ledit troisième domaine est capable de fonctionner comme matrice pour l'activité promotrice dudit deuxième domaine, dans laquelle les domaines de la construction sont orientés A-B-C ou B-C-A et dans laquelle, en outre, le troisième domaine ne contient pas de séquence cible.

(ii) l'élimination des constructions polydésoxynucléotidiques non hybridées ;
(iii) la transcription de copies multiples d'oligomères d'ARN qui sont complémentaires de la séquence matrice, c', dudit troisième domaine, C, de ladite construction polydésoxynucléotidique via l'activité d'une ARN-polymérase ADN-dépendante ; et
(iv) la détection de la quantité de transcrits d'ARN formés à l'étape (iii).

2. Test d'hybridation d'acides nucléiques selon la revendication 1 dans lequel ledit deuxième domaine, B, fournit un promoteur pour l'ARN-polymérase ADN-dépendante du bactériophage T7.

3. Test d'hybridation d'acides nucléiques selon la revendication 1 dans lequel ledit second domaine, B, fournit un promoteur pour l'ARN-polymérase ADN-dépendante du bactériophage T3.

4. Test d'hybridation d'acides nucléiques selon la revendication 1 dans lequel ledit deuxième domaine, B, fournit un promoteur pour l'ARN-polymérase ADN-dépendante du bactériophage SP6.

5. Test d'hybridation d'acides nucléiques selon l'une quelconque des revendications 1 à 4 dans lequel ledit premier domaine A de ladite construction polydésoxynucléotidique a une longueur d'au moins 10 et pas plus de 40 nucléotides.

6. Test d'hybridation d'acides nucléiques selon la revendication 5 dans lequel ledit premier domaine A a une longueur d'au moins 15 et pas plus de 30 nucléotides.

7. Test d'hybridation d'acides nucléiques selon la revendication 5 dans lequel ledit deuxième domaine B de ladite construction polydésoxynucléotidique a une longueur d'au moins 12 et pas plus de 40 nucléotides.

8. Test d'hybridation d'acides nucléiques selon la revendication 7 dans lequel ledit deuxième domaine B a une longueur d'au moins 17 et pas plus de 30 nucléotides.

9. Test d'hybridation d'acides nucléiques selon la revendication 7 dans lequel ledit troisième domaine C de ladite construction polydésoxynucléotidique a une longueur d'au moins 30 et pas plus de 10 000 nucléotides.

10. Test d'hybridation d'acides nucléiques selon la revendication 7 dans lequel ledit troisième domaine C de ladite construction polydésoxynucléotidique a une longueur d'au moins 40 et pas plus de 80 nucléotides.

11. Test d'hybridation d'acides nucléiques selon la revendication 7 dans lequel ledit troisième domaine C de ladite construction polydésoxynucléotidique a une longueur d'au moins 2 Kb et pas plus de 10 Kb.

12. Test d'hybridation d'acides nucléiques selon la revendication 7 dans lequel ledit troisième domaine C de ladite construction polydésoxynucléotidique a une longueur d'au moins 3 Kb et pas plus de 4 Kb.

13. Test d'hybridation d'acides nucléiques selon la revendication 7 dans lequel ledit troisième domaine C de ladite construction polydésoxynucléotidique est constitué de substantiellement tout le génome du virus de l'hépatite B.

14. Test d'hybridation d'acides nucléiques selon la revendication 2 dans lequel la séquence dudit deuxième domaine B comprend la séquence :

```
5'-TAA TAC GAC TCA CTA TA-3'
3'-ATT ATG CTG AGT GAT AT-5'
```

**15.** Test d'hybridation d'acides nucléiques selon la revendication 2 dans lequel la séquence nucléotidique dudit deuxième domaine B est :

```
5'-CTG GCT TAT CGA AAT TAA TAC GAC TCA CTA TA-3'
3'-GAC CGA ATA GCT TTA ATT ATG CTG AGT GAT AT-5'
```

**16.** Test d'hybridation d'acides nucléiques selon la revendication 1 dans lequel le résidu en 5' du brin supérieur de la séquence nucléotidique dudit troisième domanie C est adjacent audit deuxième domaine B et est un résidu guanosine.

**17.** Test d'hybridation d'acides nucléiques selon la revendication 1 dans lequel la séquence dudit troisième domaine C est :

```
5'-GGG AGA TGT GGT TGT CGT ACT TAG CGA AAT ACT GTC CGA
GTC G-3'
3'-CCC TCT ACA CCA ACA GCA TGA ATC GCT TTA TGA CAG GCT
CAG C-5'
```

**18.** Test d'hybridation d'acides nucléiques selon la revendication 17 dans lequel l'extrémité 3'du brin supérieur de la séquence nucléotidique d'ADN dudit deuxième domaine B est attachée à l'extrémité 5' du brin supérieur de ladite séquence nucléotidique du domaine C.

**19.** Test d'hybridation d'acides nucléiques selon la revendication 1 dans lequel le transcrit dudit troisième domaine C possède deux sous-domaines :

(a) un premier sous-domaine, $c_1$, qui est capable de s'hybrider avec un linker de capture oligonucléotidique, ledit linker de capture étant capable de s'hybrider avec un polynucléotide immobilisé sur un substrat solide ; et

(b) un second sous-domaine, $c_2$, qui est capable de se lier à un linker d'amplification oligonucléotidique, ledit linker d'amplification étant capable de se lier à une sonde quantifiable.

**20.** Test d'hybridation d'acides nucléiques selon la revendication 1 dans lequel lesdits deuxième et troisième domaines, B et C, sont présents en unités répétées multiples.

**21.** Test d'hybridation d'acides nucléiques selon la revendication 1 dans lequel ladite construction polydésoxynucléotidique est hybridée directement avec un analyte simple brin.

**22.** Test d'hybridation d'acides nucléiques selon la revendication 1 dans lequel ladite construction polydésoxynucléotidique est hybridée avec un linker oligonucléotidique, ledit linker ayant un domaine qui est capable de former des hybrides stables avec l'analyte.

**23.** Test d'hybridation d'acides nucléiques selon la revendication 1 dans lequel :

(a) ledit troisième domaine, C, de ladite construction polydésoxynucléotidique est transcrit en présence de ribonucléotides triphosphates marqués ;
(b) les transcrits dudit troisième domaine ont un premier sous-domaine, $c_1$, complémentaire d'une sonde de capture oligonucléotidique qui est immobilisée sur un substrat solide ; et
(c) lesdits transcrits sont immobilisés et quantifiés.

**24.** Test d'hybridation d'acides nucléiques selon la revendication 1 dans lequel :

(a) ledit troisième domaine, C, de ladite construction polydésoxynucléotidique est transcrit en présence de ribonucléotides triphosphates biotinylés ;
(b) ledit transcrit dudit troisième domaine a un premier sous-domaine, $c_2$, qui est complémentaire d'une sonde oligonucléotidique marquée ; et
(c) lesdits transcrits sont immobilisés sur un substrat solide avidinylé ; et
(d) lesdits transcrits sont quantifiés.

**25.** Test d'hybridation d'acides nucléiques selon la revendication 1 dans lequel :

(a) ledit troisième domaine,C, de ladite construction polydésoxynucléotidique est transcrit en présence à la fois de ribonucléotides triphosphates marqués et biotinylés ;
(b) lesdits transcrits sont immobilisés sur un substrat solide avidinylé ; et
(c) lesdits transcrits sont quantifiés.

**26.** Test d'hybridation d'acides nucléiques selon la revendication 1 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture oligonucléotidique, ladite sonde étant immobilisée sur un substrat solide ; et
(ii) un second sous-domaine, $c_2$, qui est complémentaire d'une sonde oligonucléotidique marquée ;

(b) ledit transcrit est hybridé avec ladite sonde de capture ;

(c) ladite sonde marquée est hybridée avec lesdits transcrits ; et

(d) lesdits transcrits sont quantifiés.

**27.** Test d'hybridation d'acides nucléiques selon la revendication 1 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture du transcrit, ladite sonde de capture du transcrit étant capable de s'hybrider avec un oligonucléotide qui a été immobilisé sur un substrat solide ; et

(ii) un deuxième domaine, $c_2$, qui est complémentaire d'une sonde linker, ladite sonde linker étant capable de s'hybrider avec un oligonucléotide marqué ;

(b) ledit transcrit est hybridé avec ladite sonde de capture du transcrit et avec ladite sonde linker pour former un transcrit en sandwich ;

(c) ledit transcrit en sandwich est hybridé avec un oligonucléotide immobilisé sur un substrat solide ;

(d) ledit sandwich immobilisé est hybridé avec un oligonucléotide de marquage ; et

(e) lesdits transcrits sont quantifiés.

**28.** Test d'hybridation d'acides nucléiques selon la revendication 1 dans lequel :

(a) le transcrit dudit troisième domaine, C, de ladite construction polydésoxynucléotidique présente deux sous-domaines :

(i) un premier sous-domaine, $c_1$, qui est complémentaire d'une sonde de capture du transcrit, ladite sonde de capture du transcrit étant capable de s'hybrider avec un oligonucléotide qui a été immobilisé sur un substrat solide ; et
(ii) un second sous-domaine, $c_2$, qui est complémentaire d'une sonde linker d'amplification, ladite sonde linker étant capable de s'hybrider avec une sonde d'amplification ;

(b) ledit transcrit est hybridé avec ladite sonde de capture du transcrit et ladite sonde linker d'amplification pour former un transcrit sandwich ;

(c) ledit transcrit sandwich est hybridé avec un oligonucléotide immobilisé sur un substrat solide ;

(d) ledit sandwich immobilisé est hybridé avec une sonde d'amplification ;

(e) ladite sonde d'amplification est hybridée avec un oligonucléotide de marquage ; et

(f) lesdits transcrits sont quantifiés.

**29.** Test d'hybridation d'acides nucléiques selon la revendication 28 dans lequel le transcrit d'ARN est transcrit à partir de l'ADN du virus de l'hépatite B.

**30.** Méthode selon la revendication 1 utilisée pour détecter la présence de N. gonorrhoeae dans un échantillon biologique dans lequel l'analyte comprend un segment d'ADN ou d'ARN de N. gonorrhoeae.

**31.** Méthode selon la revendication 1 utilisée pour détecter la présence du virus de l'hépatite B dans un échantillon biologique dans lequel l'analyte comprend un segment d'ADN ou d'ARN du virus de l'hépatite B.

**32.** Méthode selon la revendication 1 utilisée pour détecter la présence de bactéries contenant le gène TEM-1 de la β-lactamase, dans un échantillon biologique dans lequel l'analyte comprend un segment d'ADN ou d'ARN du gène TEM-1 de la β-lactamase.

**33.** Méthode selon la revendication 1 utilisée pour détecter la présence de Chlamydia dans un échantillon biologique dans lequel l'analyte comprend un segment d'ADN ou d'ARN de Chlamydia.

**34.** Méthode selon la revendication 1 utilisée pour détecter la présence de bactéries contenant le déterminant tet M dans un échantillon biologique dans lequel l'analyte comprend un segment d'ADN ou d'ARN du déterminant tet M.

**35.** Méthode selon la revendication 1 utilisée pour détecter la présence de HIV dans un échantillon biologique dans lequel l'analyte comprend un segment d'ADN ou d'ARN de HIV.

**36.** Méthode selon la revendication 1 utilisée pour détecter la présence de HCV dans un échantillon biologique dans lequel l'analyte comprend un segment d'ADN ou d'ARN de HCV.

**37.** Méthode d'amplification du signal utilisée pour détecter et quantifier un récepteur de ligand dans un test comprenant les étapes suivantes :

(a) immobilisation dudit récepteur de ligand directement ou indirectement sur une phase solide ;

(b) liaison au récepteur de ligand d'un ligand spécifique dudit récepteur, ledit ligand étant couplé à un oligonucléotide comme défini dans la revendication 1 ;

(c) élimination du ligand non lié ;

(d) hybridation d'une construction polydésoxynucléotidique comme défini dans la revendication 1 avec l'oligonucléotide ;

(e) élimination des constructions non hybridées ;

(f) transcription de copies multiples d'oligomères d'ARN qui sont complémentaires de la séquence matrice, c', du troisième domaine, C, de ladite construction polydésoxynucléotidique via l'activité d'une ARN-polymérase ADN-dépendante ; et

(g) quantification des transcrits d'ARN.

38. Test d'hybridation selon la revendication 37 dans lequel le récepteur de ligand est un antigène et le ligand est un anticorps qui réagit immunologiquement avec l'antigène.

39. Méthode de réalisation d'une construction polydésoxynucléotidique telle que définie à la revendication 1 qui comprend la combinaison des domaines A, B et C par des liens covalents dans l'orientation A-B-C ou B-C-A.

40. Construction polydésoxynucléotidique isolée destinée à une utilisation comme amplificateur de signal dans des tests d'hybridation comprenant trois domaines :

(a) un premier domaine (A) qui est simple brin et a une séquence nucléotidique complémentaire d'une séquence cible ;

(b) un deuxième domaine (B) qui est double brin et est capable de fonctionner comme promoteur de l'activité enzymatique d'une ARN-polymérase ADN-dépendante ; et

(c) un troisième domaine (C) qui est soit simple brin soit double brin et adjacent audit deuxième domaine, de sorte que ledit troisième domaine est capable de fonctionner comme matrice pour l'activité promotrice dudit deuxième domaine, dans laquelle les domaines de la construction sont orientés A-B-C ou B-C-A et dans laquelle, en outre, le troisième domaine ne contient pas de séquence cible.

## Figure 1-1

A -
$$\text{---- A ---|--- B ----|--- C ----}$$

```
5' —                    ┌——b——┬——— c ———AAA
                                                A
3' — ——a'———┴——b'———┴——— c'———AAA
```

B -     <              A              >< B              (T7 PROMOTER)       >...
5'-                                   CTGGCTTATCGAAATTAATACGACTCACTATA...
3'-AATCCGTATCCTGGGCACAGGACCGAATAGCTTTAATTATGCTGAGTGATAT...

<              C              (TRANSCRIBED REGION)       >
...GGGAGATGTGGTTGTCGTACTTAGCGAAATACTGTCCGAGTCGAAAA┐
...CCCTCTACACCAACAGCATGAATCGCTTTATGACAGGCTCAGCAAA ┘
               template strand

EP 0 510 085 B1

Figure 1-2

EP 0 510 085 B1

# Figure 2-1

A -

microtiter
dish

analyte capture

template linker

—w——⊤——y—        —x——⊤——a——

w'——    —y'——⊤—— x'—    —a'———— b'——⊤——c'—

analyte

template probe

## Figure 2-2

B -

Figure 2-3

C -

EP 0 510 085 B1

```
                    Transcript
                  Capture Probe                              Label Probe

Solid       5' -<              C'₂               >
Substrate---XCGACTCGGACAGTATTTCGC<                      C'₁           >-3'
                                        TAAGTACGACAACCACATC-------Label

          3' - GCTGAGCCTGTCATAAAGCGATTCATGCTGTTGGTGTAGAGGG-5'
               <              C₂            ><              C₁            >
               --------------------------------Transcript----------------------------------
```

Figure 3A

Figure 3B

Figure 3C

pII$_R$

EP 0 510 085 B1

Figure 4    X    5' ——————————— 5'

Y   3' ———————————————— 3'

|——————— A ———————|

OLIGO-N

X - 5'- GGTCGACTAATCGGTAGC

Y - 3'- TCCGTATCCTGGGCACAGCCAGCTGATTAGCCATCGAT


OLIGO-S

X - 5'- GGTCGACTAATCGGTAGC

Y'- 3'- TCCGTATCCTGGGCACAGCCAGCTGATTAGCCATCG


OLIGO-H

X - 5'- GGTCGACTAATCGGTAGC

Y"- 3'- TCCGTATCCTGGGCACAGCCAGCTGATTAGCCATCGTCGA

Figure 5

EP 0 510 085 B1

STD. vs. P11 Probes
T7 Assay

Figure 6

signal

analyte concentration (amol)

STD
P11
P11-L
P11-R